# EUROPEAN PATENT SPECIFICATION

(11) **EP 2 954 070 B1**
(45) Date of publication and mention of the grant of the patent: **01.04.2020**
(21) Application number: 14749095.7
(22) Date of filing: 11.02.2014
(51) Int. Cl.: C12Q 1/6869, C12Q 1/6883

(54) **METHOD FOR EVALUATING AN IMMUNOREPERTOIRE**
VERFAHREN ZUR BEURTEILUNG EINES IMMUNREPERTOIRES
PROCÉDÉ D'ÉVALUATION D'UN IMMUNORÉPERTOIRE

(30) Priority: 11.02.2013 US 201361763451 P
(43) Date of publication of application: 16.12.2015
(73) Proprietor: iRepertoire, Inc., Huntsville, AL 35806 (US)
(72) Inventor: WANG, Chunlin, Menlo Park, CA 94025 (US); HAN, Jian, Huntsville, AL 35802 (US)
(74) Representative: Dehns
(86) International application number: PCT/US2014/015841
(87) International publication number: WO 2014/124451

(56) References cited:
- WO-A1-2011/139371
- WO-A1-2012/097374
- WO-A1-2013/049727
- WO-A1-2015/075939
- WO-A2-2011/140433
- US-A1- 2002 110 807
- US-A1- 2010 021 896
- US-A1- 2011 207 135
- US-A1- 2012 171 725
- HE J ET AL: "IgH gene rearrangements as plasma biomarkers in Non-Hodgkin's Lymphoma patients", ONCOTARGET, vol. 2, no. 3, 1 January 2011 (2011-01-01) , pages 178-185, XP055247543, DOI: 10.18632/oncotarget.235
- FREEMAN D J ET AL: "Profiling the T-cell receptor beta-chain repertoire by massively parallel sequencing", GENOME RESEARCH, COLD SPRING HARBOR LABORATORY PRESS, US, vol. 19, no. 10, 1 October 2009 (2009-10-01), pages 1817-1824, XP002636496, ISSN: 1088-9051, DOI: 10.1101/GR.092924.109 [retrieved on 2009-06-18]
- WANG, C ET AL.: 'High Throughput Sequencing Reveals A Complex Pattem Of Dynamic Interrelationships Among Human T Cell Subsets.' PNAS vol. 107, 26 January 2010, pages 1518 - 1523, XP055114155

### SEQUENCE LISTING

<110> CB BIOTECHNOLOGIES, INC.
<120> METHOD FOR EVALUATING AN IMMUNOREPERTOIRE
<130> 15892-0005
<140>
   <141>
<150> 61/763,451
   <151> 2013-02-11
<160> 358
<170> PatentIn version 3.5
<210> 1
   <211> 220
   <212> DNA
   <213> Artificial Sequence
<220>
   <223> Description of Artificial Sequence: Synthetic polynucleotide
<220>
   <221> modified_base
   <222> (1)..(10)
   <223> a, c, t, g, unknown or other
<220>
   <221> modified_base
   <222> (12)..(12)
   <223> a, c, t, g, unknown or other
<220>
   <221> modified_base
   <222> (15)..(15)
   <223> a, c, t, g, unknown or other
<220>
   <221> modified_base
   <222> (31)..(31)
   <223> a, c, t, g, unknown or other
<220>
   <221> modified_base
   <222> (33)..(33)
   <223> a, c, t, g, unknown or other
<220>
   <221> modified_base
   <222> (98)..(98)
   <223> a, c, t, g, unknown or other
<220>
   <221> modified_base
   <222> (103)..(103)
   <223> a, c, t, g, unknown or other
<220>
   <221> modified_base
   <222> (212)..(212)
   <223> a, c, t, g, unknown or other
<220>
   <221> modified_base
   <222> (215)..(215)
   <223> a, c, t, g, unknown or other
<220>
   <221> modified_base
   <222> (218)..(218)
   <223> a, c, t, g, unknown or other
<400> 1
   nnnnnnnnnn cntantcggt ctaagggtac ngntacctcc ttttgaagga gctccagatg 60
   aaagactctg cctcttacct ctgtgctgtg agagatanca acnatcactt aatcttgggc 120
   gctgggagca gactaattat aatgccagat atccacaacc ctgaccctgc cgcgtaccag 180
   ctgaaagact atgaacagga tggggaggca gnagnagnag 220
<210> 2
   <211> 180
   <212> DNA
   <213> Artificial Sequence
<220>
   <223> Description of Artificial Sequence: Synthetic polynucleotide
<220>
   <221> modified_base
   <222> (1)..(10)
   <223> a, c, t, g, unknown or other
<220>
   <221> modified_base
   <222> (12)..(12)
   <223> a, c, t, g, unknown or other
<220>
   <221> modified_base
   <222> (14)..(14)
   <223> a, c, t, g, unknown or other
<220>
   <221> modified_base
   <222> (16)..(17)
   <223> a, c, t, g, unknown or other
<220>
   <221> modified_base
   <222> (40)..(40)
   <223> a, c, t, g, unknown or other
<400> 2
   nnnnnnnnnn gnangnncag ggttctggat atttggtttn acaattagct tggtccctgc 60
   tccaaagatt aatttgtagt tgctatccct cacagcacag aggtaagagg aagagtattt 120
   cttctggagc tccttcaaca ggaggaaact gtacccttta tacctactaa ggaatgaaga 180
<210> 3
   <211> 212
   <212> DNA
   <213> Artificial Sequence
<220>
   <223> Description of Artificial Sequence: Synthetic polynucleotide
<220>
   <221> modified_base
   <222> (1)..(12)
   <223> a, c, t, g, unknown or other
<220>
   <221> modified_base
   <222> (14)..(15)
   <223> a, c, t, g, unknown or other
<220>
   <221> modified_base
   <222> (26)..(27)
   <223> a, c, t, g, unknown or other
<220>
   <221> modified_base
   <222> (85)..(85)
   <223> a, c, t, g, unknown or other
<220>
   <221> modified_base
   <222> (87)..(88)
   <223> a, c, t, g, unknown or other
<220>
   <221> modified_base
   <222> (90)..(90)
   <223> a, c, t, g, unknown or other
<220>
   <221> modified_base
   <222> (95)..(96)
   <223> a, c, t, g, unknown or other
<220>
   <221> modified_base
   <222> (109)..(109)
   <223> a, c, t, g, unknown or other
<220>
   <221> modified_base
   <222> (123)..(123)
   <223> a, c, t, g, unknown or other
<220>
   <221> modified_base
   <222> (132)..(133)
   <223> a, c, t, g, unknown or other
<220>
   <221> modified_base
   <222> (141)..(141)
   <223> a, c, t, g, unknown or other
<220>
   <221> modified_base
   <222> (144)..(144)
   <223> a, c, t, g, unknown or other
<220>
   <221> modified_base
   <222> (149)..(149)
   <223> a, c, t, g, unknown or other
<220>
   <221> modified_base
   <222> (153)..(153)
   <223> a, c, t, g, unknown or other
<220>
   <221> modified_base
   <222> (172)..(172)
   <223> a, c, t, g, unknown or other
<220>
   <221> modified_base
   <222> (180)..(180)
   <223> a, c, t, g, unknown or other
<220>
   <221> modified_base
   <222> (188)..(190)
   <223> a, c, t, g, unknown or other
<220>
   <221> modified_base
   <222> (206)..(206)
   <223> a, c, t, g, unknown or other
<220>
   <221> modified_base
   <222> (208)..(212)
   <223> a, c, t, g, unknown or other
<400> 3
   nnnnnnnnnn nntnncggtt ctcttnntcg ctgctcatcc tccaggtgcg ggaggcagat 60
   gctgctgttt actactgtgc tgtgnannan ggcanngaca acaacctcnt ctttggtgga 120
   ggnaccctac tnntggttat nccnaatanc canaaccctg accctgccga gnagcagcan 180
   aaaaactnnn aggggggtgg agaagnannn nn 212
<210> 4
   <211> 257
   <212> DNA
   <213> Artificial Sequence
<220>
   <223> Description of Artificial Sequence: Synthetic polynucleotide
<220>
   <221> modified_base
   <222> (1)..(14)
   <223> a, c, t, g, unknown or other
<220>
   <221> modified_base
   <222> (17)..(19)
   <223> a, c, t, g, unknown or other
<220>
   <221> modified_base
   <222> (22)..(22)
   <223> a, c, t, g, unknown or other
<220>
   <221> modified_base
   <222> (252)..(252)
   <223> a, c, t, g, unknown or other
<220>
   <221> modified_base
   <222> (256)..(256)
   <223> a, c, t, g, unknown or other
<400> 4
   nnnnnnnnnn nnnnggnnng gnagctatgg ctttgaagct gaatttaaca agagccaaac 60
   ctccttccac ctgaagaaac catctgccct tgtgagcgac tccgctttgt acttctgtgc 120
   tgtgagagac atcaacgctg ccggcaacaa cctaactttt ggaggaagaa ccatggtgct 180
   agttaaacca aatatccata accctgacgc tgccgtgtac cagctgaaag actctgaggg 240
   ggctggagag gnaggng 257
<210> 5
   <211> 236
   <212> DNA
   <213> Artificial Sequence
<220>
   <223> Description of Artificial Sequence: Synthetic polynucleotide
<220>
   <221> modified_base
   <222> (1)..(5)
   <223> a, c, t, g, unknown or other
<220>
   <221> modified_base
   <222> (7)..(8)
   <223> a, c, t, g, unknown or other
<220>
   <221> modified_base
   <222> (11)..(15)
   <223> a, c, t, g, unknown or other
<220>
   <221> modified_base
   <222> (166)..(166)
   <223> a, c, t, g, unknown or other
<220>
   <221> modified_base
   <222> (194)..(194)
   <223> a, c, t, g, unknown or other
<220>
   <221> modified_base
   <222> (223)..(223)
   <223> a, c, t, g, unknown or other
<220>
   <221> modified_base
   <222> (225)..(225)
   <223> a, c, t, g, unknown or other
<220>
   <221> modified_base
   <222> (228)..(228)
   <223> a, c, t, g, unknown or other
<220>
   <221> modified_base
   <222> (231)..(231)
   <223> a, c, t, g, unknown or other
<220>
   <221> modified_base
   <222> (233)..(236)
   <223> a, c, t, g, unknown or other
<400> 5
   nnnnnanngg nnnnngttta tccctgccga cagaaagtcc agcactctga gcctgccccg       60
   ggtttccctg agcgacactg ctgtgtacta ctgcctcgtg ggtgaccggt ctggaaacag     120
   cgatgaaatt ttcatcttag gaagaagaac gcttctagtc atccanccca acatccacaa     180
   ccctgccgcg gagnagcacc agaaaaaaga tgatgagggg gangnagnag nannnn          236
<210> 6
   <211> 241
   <212> DNA
   <213> Artificial Sequence
<220>
   <223> Description of Artificial Sequence: Synthetic polynucleotide
<220>
   <221> modified_base
   <222> (1)..(16)
   <223> a, c, t, g, unknown or other
<220>
   <221> modified_base
   <222> (19)..(19)
   <223> a, c, t, g, unknown or other
<220>
   <221> modified_base
   <222> (23)..(23)
   <223> a, c, t, g, unknown or other
<220>
   <221> modified_base
   <222> (151)..(151)
   <223> a, c, t, g, unknown or other
<220>
   <221> modified_base
   <222> (173)..(173)
   <223> a, c, t, g, unknown or other
<220>
   <221> modified_base
   <222> (187)..(187)
   <223> a, c, t, g, unknown or other
<220>
   <221> modified_base
   <222> (199)..(199)
   <223> a, c, t, g, unknown or other
<220>
   <221> modified_base
   <222> (233)..(233)
   <223> a, c, t, g, unknown or other
<220>
   <221> modified_base
   <222> (239)..(239)
   <223> a, c, t, g, unknown or other
<220>
   <221> modified_base
   <222> (241)..(241)
   <223> a, c, t, g, unknown or other
<400> 6
   nnnnnnnnnn nnnnnntcnc tgntctattg aataaaaagg ataaacatct gtctctgcgc     60
   attgcagaca cccagactgg ggactcagct atctacttct gtgcagagag ccccggtggc    120
   ggcagcaact tcttctttgg tggaggagca ntactactag tcgttctaca tanccacaac    180
   catgatnccg ccgagtacnt gctgaaaaaa tatgatgagg atggagaaga agnagcatna    240
   n                                                                    241
<210> 7
   <211> 237
   <212> DNA
   <213> Artificial Sequence
<220>
   <223> Description of Artificial Sequence: Synthetic polynucleotide
<220>
   <221> modified_base
   <222> (1)..(8)
   <223> a, c, t, g, unknown or other
<220>
   <221> modified_base
   <222> (18)..(19)
   <223> a, c, t, g, unknown or other
<220>
   <221> modified_base
   <222> (132)..(132)
   <223> a, c, t, g, unknown or other
<220>
   <221> modified_base
   <222> (152)..(153)
   <223> a, c, t, g, unknown or other
<220>
   <221> modified_base
   <222> (191)..(192)
   <223> a, c, t, g, unknown or other
<220>
   <221> modified_base
   <222> (194)..(194)
   <223> a, c, t, g, unknown or other
<220>
   <221> modified_base
   <222> (202)..(202)
   <223> a, c, t, g, unknown or other
<220>
   <221> modified_base
   <222> (205)..(205)
   <223> a, c, t, g, unknown or other
<220>
   <221> modified_base
   <222> (207)..(207)
   <223> a, c, t, g, unknown or other
<220>
   <221> modified_base
   <222> (209)..(209)
   <223> a, c, t, g, unknown or other
<220>
   <221> modified_base
   <222> (235)..(237)
   <223> a, c, t, g, unknown or other
<400> 7
   nnnnnnnnct gagggtannc gtctctcggg agaagaagga atcctttcct ctcactgtga      60
   catcggccca aaagaacccg acagctttct atctctgtgc cagtagtatg gggggggggg     120
   cctacaatga gnacggcggc gggggaggga cnntgctcgt cgtggaggag gacatgaagg     180
   tcttgcccgc nncngaggaa gntgnanang aaccataaaa atgcgctggc tgaannn        237
<210> 8
   <211> 280
   <212> DNA
   <213> Artificial Sequence
<220>
   <223> Description of Artificial Sequence: Synthetic polynucleotide
<220>
   <221> modified_base
   <222> (1)..(11)
   <223> a, c, t, g, unknown or other
<220>
   <221> modified_base
   <222> (16)..(21)
   <223> a, c, t, g, unknown or other
<220>
   <221> modified_base
   <222> (23)..(23)
   <223> a, c, t, g, unknown or other
<220>
   <221> modified_base
   <222> (214)..(214)
   <223> a, c, t, g, unknown or other
<220>
   <221> modified_base
   <222> (245)..(245)
   <223> a, c, t, g, unknown or other
<220>
   <221> modified_base
   <222> (247)..(248)
   <223> a, c, t, g, unknown or other
<220>
   <221> modified_base
   <222> (250)..(250)
   <223> a, c, t, g, unknown or other
<220>
   <221> modified_base
   <222> (261)..(261)
   <223> a, c, t, g, unknown or other
<220>
   <221> modified_base
   <222> (270)..(270)
   <223> a, c, t, g, unknown or other
<220>
   <221> modified_base
   <222> (273)..(273)
   <223> a, c, t, g, unknown or other
<220>
   <221> modified_base
   <222> (276)..(276)
   <223> a, c, t, g, unknown or other
<220>
   <221> modified_base
   <222> (280)..(280)
   <223> a, c, t, g, unknown or other
<400> 8
   nnnnnnnnnn ngctcnnnnn ncncatacga gcaaggcgtc gagaaggaca agtttctcac      60
   aaccatgcaa gcctgacctt gtccactctg acagtgacca gtgcccatcc tgaagacagc     120
   agcttctaca tctgcagtgc tagagggggg gggggggacg actactacta cttcggcggg     180
   gggggcatgc tgatcgtgga ggaggaggac atgnagctcc tccccgccgc cgaggttgtt     240
   gtgtntnnan catcatactg ntggtggagn agnagnagcn                           280
<210> 9
   <211> 172
   <212> DNA
   <213> Artificial Sequence
<220>
   <223> Description of Artificial Sequence: Synthetic polynucleotide
<220>
   <221> modified_base
   <222> (1)..(15)
   <223> a, c, t, g, unknown or other
<220>
   <221> modified_base
   <222> (17)..(27)
   <223> a, c, t, g, unknown or other
<220>
   <221> modified_base
   <222> (35)..(35)
   <223> a, c, t, g, unknown or other
<400> 9
   nnnnnnnnnn nnnnngnnnn nnnnnnntac tttcngaatg aagaacttat tcagaaagca      60
   gaaataatca atgagcgatt tttagcccaa tgctccaaaa actcatcctg taccttggag     120
   ttccagtcca cggagtcagg ggacacagca ctgtatttct gtgccagcag ca             172
<210> 10
   <211> 278
   <212> DNA
   <213> Artificial Sequence
<220>
   <223> Description of Artificial Sequence: Synthetic polynucleotide
<220>
   <221> modified_base
   <222> (1)..(3)
   <223> a, c, t, g, unknown or other
<220>
   <221> modified_base
   <222> (5)..(11)
   <223> a, c, t, g, unknown or other
<220>
   <221> modified_base
   <222> (13)..(14)
   <223> a, c, t, g, unknown or other
<220>
   <221> modified_base
   <222> (18)..(18)
   <223> a, c, t, g, unknown or other
<220>
   <221> modified_base
   <222> (20)..(20)
   <223> a, c, t, g, unknown or other
<220>
   <221> modified_base
   <222> (252)..(252)
   <223> a, c, t, g, unknown or other
<220>
   <221> modified_base
   <222> (256)..(256)
   <223> a, c, t, g, unknown or other
<220>
   <221> modified_base
   <222> (263)..(266)
   <223> a, c, t, g, unknown or other
<220>
   <221> modified_base
   <222> (268)..(268)
   <223> a, c, t, g, unknown or other
<220>
   <221> modified_base
   <222> (270)..(271)
   <223> a, c, t, g, unknown or other
<220>
   <221> modified_base
   <222> (273)..(273)
   <223> a, c, t, g, unknown or other
<220>
   <221> modified_base
   <222> (277)..(278)
   <223> a, c, t, g, unknown or other
<400> 10
   nnngnnnnnn nanngganan gcacaagaag cgattctcat ctcaatgccc caagaacgca 60
   ccctgcagcc tggcaatcct gtcctcagaa ccgggagaca cggcactgta tctctgcgcc 120
   agcagtcaat cggggggggg ggggagggcc gtccgcagcg gggggggggg gggccggggg 180
   acggtcccaa agagaaagaa aacctgcccc ccgcgctcgg gcggtgtgat tgagcgaaac 240
   agacaggaag gnaagnaaaa aannnnancn ncnctcnn 278
<210> 11
   <211> 253
   <212> DNA
   <213> Artificial Sequence
<220>
   <223> Description of Artificial Sequence: Synthetic polynucleotide
<220>
   <221> modified_base
   <222> (1)..(8)
   <223> a, c, t, g, unknown or other
<220>
   <221> modified_base
   <222> (10)..(11)
   <223> a, c, t, g, unknown or other
<220>
   <221> modified_base
   <222> (13)..(15)
   <223> a, c, t, g, unknown or other
<220>
   <221> modified_base
   <222> (25)..(25)
   <223> a, c, t, g, unknown or other
<220>
   <221> modified_base
   <222> (120)..(120)
   <223> a, c, t, g, unknown or other
<220>
   <221> modified_base
   <222> (234)..(235)
   <223> a, c, t, g, unknown or other
<220>
   <221> modified_base
   <222> (238)..(238)
   <223> a, c, t, g, unknown or other
<220>
   <221> modified_base
   <222> (243)..(243)
   <223> a, c, t, g, unknown or other
<220>
   <221> modified_base
   <222> (246)..(246)
   <223> a, c, t, g, unknown or other
<220>
   <221> modified_base
   <222> (249)..(249)
   <223> a, c, t, g, unknown or other
<220>
   <221> modified_base
   <222> (252)..(253)
   <223> a, c, t, g, unknown or other
<400> 11
   nnnnnnnngn nannntctga tgganacagt gtctctcgac aggcacaggc taaattctcc 60
   ctgtccctag agtctgccat ccccaaccag acagctcttt acttctgtgc caccagtgan 120
   gcggggggcg gggaccacta cttcgggggg gggaggcgga ccagggtgct ggtcgacgag 180
   aaaaaggagc tcccccccgc cgccgctgtg gttgttgctt cataataatc aggnnggnga 240
   ggnagnagna ann 253
<210> 12
   <211> 20
   <212> DNA
   <213> Artificial Sequence
<220>
   <223> Description of Artificial Sequence: Synthetic primer
<400> 12
   tgcacgtacc agacatctgg 20
<210> 13
   <211> 20
   <212> DNA
   <213> Artificial Sequence
<220>
   <223> Description of Artificial Sequence: Synthetic primer
<400> 13
   aggtcgtttt tcttcattcc 20
<210> 14
   <211> 39
   <212> DNA
   <213> Artificial Sequence
<220>
   <223> Description of Artificial Sequence: Synthetic primer
<400> 14
   gcctccctcg cgccatcaga ggtcgttttt cttcattcc 39
<210> 15
   <211> 20
   <212> DNA
   <213> Artificial Sequence
<220>
   <223> Description of Artificial Sequence: Synthetic primer
<400> 15
   tctgtaatca ctctgtgtcc 20
<210> 16
   <211> 20
   <212> DNA
   <213> Artificial Sequence
<220>
   <223> Description of Artificial Sequence: Synthetic primer
<400> 16
   agggacgata caacatgacc 20
<210> 17
   <211> 39
   <212> DNA
   <213> Artificial Sequence
<220>
   <223> Description of Artificial Sequence: Synthetic primer
<400> 17
   gcctccctcg cgccatcaga gggacgatac aacatgacc 39
<210> 18
   <211> 20
   <212> DNA
   <213> Artificial Sequence
<220>
   <223> Description of Artificial Sequence: Synthetic primer
<400> 18
   ctattcagtc tctggaaacc 20
<210> 19
   <211> 20
   <212> DNA
   <213> Artificial Sequence
<220>
   <223> Description of Artificial Sequence: Synthetic primer
<400> 19
   atacatcaca ggggataacc 20
<210> 20
   <211> 39
   <212> DNA
   <213> Artificial Sequence
<220>
   <223> Description of Artificial Sequence: Synthetic primer
<400> 20
   gcctccctcg cgccatcaga tacatcacag gggataacc 39
<210> 21
   <211> 20
   <212> DNA
   <213> Artificial Sequence
<220>
   <223> Description of Artificial Sequence: Synthetic primer
<400> 21
   tgtagccaca acaacattgc 20
<210> 22
   <211> 20
   <212> DNA
   <213> Artificial Sequence
<220>
   <223> Description of Artificial Sequence: Synthetic primer
<400> 22
   aaagttacaa acgaagtggc 20
<210> 23
   <211> 39
   <212> DNA
   <213> Artificial Sequence
<220>
   <223> Description of Artificial Sequence: Synthetic primer
<400> 23
   gcctccctcg cgccatcaga aagttacaaa cgaagtggc 39
<210> 24
   <211> 20
   <212> DNA
   <213> Artificial Sequence
<220>
   <223> Description of Artificial Sequence: Synthetic primer
<400> 24
   gcacttacac agacagctcc 20
<210> 25
   <211> 20
   <212> DNA
   <213> Artificial Sequence
<220>
   <223> Description of Artificial Sequence: Synthetic primer
<400> 25
   tatggacatg aaacaagacc 20
<210> 26
   <211> 39
   <212> DNA
   <213> Artificial Sequence
<220>
   <223> Description of Artificial Sequence: Synthetic primer
<400> 26
   gcctccctcg cgccatcagt atggacatga aacaagacc 39
<210> 27
   <211> 20
   <212> DNA
   <213> Artificial Sequence
<220>
   <223> Description of Artificial Sequence: Synthetic primer
<400> 27
   gcaactatac aaactattcc 20
<210> 28
   <211> 20
   <212> DNA
   <213> Artificial Sequence
<220>
   <223> Description of Artificial Sequence: Synthetic primer
<400> 28
   gttttcttgc tactcatacg 20
<210> 29
   <211> 39
   <212> DNA
   <213> Artificial Sequence
<220>
   <223> Description of Artificial Sequence: Synthetic primer
<400> 29
   gcctccctcg cgccatcagg ttttcttgct actcatacg 39
<210> 30
   <211> 20
   <212> DNA
   <213> Artificial Sequence
<220>
   <223> Description of Artificial Sequence: Synthetic primer
<400> 30
   tgcacgtact ctgtcagtcg 20
<210> 31
   <211> 20
   <212> DNA
   <213> Artificial Sequence
<220>
   <223> Description of Artificial Sequence: Synthetic primer
<400> 31
   ggatatgaga agcagaaagg 20
<210> 32
   <211> 39
   <212> DNA
   <213> Artificial Sequence
<220>
   <223> Description of Artificial Sequence: Synthetic primer
<400> 32
   gcctccctcg cgccatcagg gatatgagaa gcagaaagg 39
<210> 33
   <211> 20
   <212> DNA
   <213> Artificial Sequence
<220>
   <223> Description of Artificial Sequence: Synthetic primer
<400> 33
   aatctcttct ggtatgtsca 20
<210> 34
   <211> 19
   <212> DNA
   <213> Artificial Sequence
<220>
   <223> Description of Artificial Sequence: Synthetic primer
<400> 34
   ggytttgagg ctgaattta 19
<210> 35
   <211> 38
   <212> DNA
   <213> Artificial Sequence
<220>
   <223> Description of Artificial Sequence: Synthetic primer
<400> 35
   gcctccctcg cgccatcagg gytttgaggc tgaattta 38
<210> 36
   <211> 20
   <212> DNA
   <213> Artificial Sequence
<220>
   <223> Description of Artificial Sequence: Synthetic primer
<400> 36
   gtccaatatc ctggagaagg 20
<210> 37
   <211> 20
   <212> DNA
   <213> Artificial Sequence
<220>
   <223> Description of Artificial Sequence: Synthetic primer
<400> 37
   aaccacttct ttccacttgg 20
<210> 38
   <211> 39
   <212> DNA
   <213> Artificial Sequence
<220>
   <223> Description of Artificial Sequence: Synthetic primer
<400> 38
   gcctccctcg cgccatcaga accacttctt tccacttgg 39
<210> 39
   <211> 20
   <212> DNA
   <213> Artificial Sequence
<220>
   <223> Description of Artificial Sequence: Synthetic primer
<400> 39
   aatgcaatta tacagtgagc 20
<210> 40
   <211> 20
   <212> DNA
   <213> Artificial Sequence
<220>
   <223> Description of Artificial Sequence: Synthetic primer
<400> 40
   tgagaacaca aagtcgaacg 20
<210> 41
   <211> 39
   <212> DNA
   <213> Artificial Sequence
<220>
   <223> Description of Artificial Sequence: Synthetic primer
<400> 41
   gcctccctcg cgccatcagt gagaacacaa agtcgaacg 39
<210> 42
   <211> 20
   <212> DNA
   <213> Artificial Sequence
<220>
   <223> Description of Artificial Sequence: Synthetic primer
<400> 42
   tcttaattgt acttatcagg 20
<210> 43
   <211> 20
   <212> DNA
   <213> Artificial Sequence
<220>
   <223> Description of Artificial Sequence: Synthetic primer
<400> 43
   tcaatcaagc cagaaggagc 20
<210> 44
   <211> 39
   <212> DNA
   <213> Artificial Sequence
<220>
   <223> Description of Artificial Sequence: Synthetic primer
<400> 44
   gcctccctcg cgccatcagt caatcaagcc agaaggagc 39
<210> 45
   <211> 20
   <212> DNA
   <213> Artificial Sequence
<220>
   <223> Description of Artificial Sequence: Synthetic primer
<400> 45
   tcagtgttcc agagggagcc 20
<210> 46
   <211> 20
   <212> DNA
   <213> Artificial Sequence
<220>
   <223> Description of Artificial Sequence: Synthetic primer
<400> 46
   atggaaggtt tacagcacag 20
<210> 47
   <211> 39
   <212> DNA
   <213> Artificial Sequence
<220>
   <223> Description of Artificial Sequence: Synthetic primer
<400> 47
   gcctccctcg cgccatcaga tggaaggttt acagcacag 39
<210> 48
   <211> 20
   <212> DNA
   <213> Artificial Sequence
<220>
   <223> Description of Artificial Sequence: Synthetic primer
<400> 48
   accctgagtg tccaggaggg 20
<210> 49
   <211> 20
   <212> DNA
   <213> Artificial Sequence
<220>
   <223> Description of Artificial Sequence: Synthetic primer
<400> 49
   ttatagacat tcgttcaaat 20
<210> 50
   <211> 39
   <212> DNA
   <213> Artificial Sequence
<220>
   <223> Description of Artificial Sequence: Synthetic primer
<400> 50
   gcctccctcg cgccatcagt tatagacatt cgttcaaat 39
<210> 51
   <211> 20
   <212> DNA
   <213> Artificial Sequence
<220>
   <223> Description of Artificial Sequence: Synthetic primer
<400> 51
   tggactgcac atatgacacc 20
<210> 52
   <211> 20
   <212> DNA
   <213> Artificial Sequence
<220>
   <223> Description of Artificial Sequence: Synthetic primer
<400> 52
   cagcaaaatg caacagaagg 20
<210> 53
   <211> 39
   <212> DNA
   <213> Artificial Sequence
<220>
   <223> Description of Artificial Sequence: Synthetic primer
<400> 53
   gcctccctcg cgccatcagc agcaaaatgc aacagaagg 39
<210> 54
   <211> 20
   <212> DNA
   <213> Artificial Sequence
<220>
   <223> Description of Artificial Sequence: Synthetic primer
<400> 54
   agctgaagtg caactattcc 20
<210> 55
   <211> 20
   <212> DNA
   <213> Artificial Sequence
<220>
   <223> Description of Artificial Sequence: Synthetic primer
<400> 55
   tctagagaga gcatcaaagg 20
<210> 56
   <211> 39
   <212> DNA
   <213> Artificial Sequence
<220>
   <223> Description of Artificial Sequence: Synthetic primer
<400> 56
   gcctccctcg cgccatcagt ctagagagag catcaaagg 39
<210> 57
   <211> 20
   <212> DNA
   <213> Artificial Sequence
<220>
   <223> Description of Artificial Sequence: Synthetic primer
<400> 57
   aatgccacca tgaactgcag 20
<210> 58
   <211> 20
   <212> DNA
   <213> Artificial Sequence
<220>
   <223> Description of Artificial Sequence: Synthetic primer
<400> 58
   gaaagagaga aacacagtgg 20
<210> 59
   <211> 39
   <212> DNA
   <213> Artificial Sequence
<220>
   <223> Description of Artificial Sequence: Synthetic primer
<400> 59
   gcctccctcg cgccatcagg aaagagagaa acacagtgg 39
<210> 60
   <211> 20
   <212> DNA
   <213> Artificial Sequence
<220>
   <223> Description of Artificial Sequence: Synthetic primer
<400> 60
   gctctgacat taaactgcac 20
<210> 61
   <211> 20
   <212> DNA
   <213> Artificial Sequence
<220>
   <223> Description of Artificial Sequence: Synthetic primer
<400> 61
   caggagacgg acagcagagg 20
<210> 62
   <211> 39
   <212> DNA
   <213> Artificial Sequence
<220>
   <223> Description of Artificial Sequence: Synthetic primer
<400> 62
   gcctccctcg cgccatcagc aggagacgga cagcagagg 39
<210> 63
   <211> 20
   <212> DNA
   <213> Artificial Sequence
<220>
   <223> Description of Artificial Sequence: Synthetic primer
<400> 63
   atgtgacctt ggactgtgtg 20
<210> 64
   <211> 20
   <212> DNA
   <213> Artificial Sequence
<220>
   <223> Description of Artificial Sequence: Synthetic primer
<400> 64
   gagcaaaatg aaataagtgg 20
<210> 65
   <211> 39
   <212> DNA
   <213> Artificial Sequence
<220>
   <223> Description of Artificial Sequence: Synthetic primer
<400> 65
   gcctccctcg cgccatcagg agcaaaatga aataagtgg 39
<210> 66
   <211> 20
   <212> DNA
   <213> Artificial Sequence
<220>
   <223> Description of Artificial Sequence: Synthetic primer
<400> 66
   actgcagtta cacagtcagc 20
<210> 67
   <211> 20
   <212> DNA
   <213> Artificial Sequence
<220>
   <223> Description of Artificial Sequence: Synthetic primer
<400> 67
   agaaagaaag gctaaaagcc 20
<210> 68
   <211> 39
   <212> DNA
   <213> Artificial Sequence
<220>
   <223> Description of Artificial Sequence: Synthetic primer
<400> 68
   gcctccctcg cgccatcaga gaaagaaagg ctaaaagcc 39
<210> 69
   <211> 20
   <212> DNA
   <213> Artificial Sequence
<220>
   <223> Description of Artificial Sequence: Synthetic primer
<400> 69
   actgcagttt cactgatagc 20
<210> 70
   <211> 20
   <212> DNA
   <213> Artificial Sequence
<220>
   <223> Description of Artificial Sequence: Synthetic primer
<400> 70
   caagtggaag acttaatgcc 20
<210> 71
   <211> 39
   <212> DNA
   <213> Artificial Sequence
<220>
   <223> Description of Artificial Sequence: Synthetic primer
<400> 71
   gcctccctcg cgccatcagc aagtggaaga cttaatgcc 39
<210> 72
   <211> 20
   <212> DNA
   <213> Artificial Sequence
<220>
   <223> Description of Artificial Sequence: Synthetic primer
<400> 72
   gggagccaat tccacgctgc 20
<210> 73
   <211> 20
   <212> DNA
   <213> Artificial Sequence
<220>
   <223> Description of Artificial Sequence: Synthetic primer
<400> 73
   atggaagatt aagcgccacg 20
<210> 74
   <211> 39
   <212> DNA
   <213> Artificial Sequence
<220>
   <223> Description of Artificial Sequence: Synthetic primer
<400> 74
   gcctccctcg cgccatcaga tggaagatta agcgccacg 39
<210> 75
   <211> 20
   <212> DNA
   <213> Artificial Sequence
<220>
   <223> Description of Artificial Sequence: Synthetic primer
<400> 75
   atttcaatta taaactgtgc 20
<210> 76
   <211> 20
   <212> DNA
   <213> Artificial Sequence
<220>
   <223> Description of Artificial Sequence: Synthetic primer
<400> 76
   aaggaagatt cacaatctcc 20
<210> 77
   <211> 39
   <212> DNA
   <213> Artificial Sequence
<220>
   <223> Description of Artificial Sequence: Synthetic primer
<400> 77
   gcctccctcg cgccatcaga aggaagattc acaatctcc 39
<210> 78
   <211> 20
   <212> DNA
   <213> Artificial Sequence
<220>
   <223> Description of Artificial Sequence: Synthetic primer
<400> 78
   gcaccaattt cacctgcagc 20
<210> 79
   <211> 20
   <212> DNA
   <213> Artificial Sequence
<220>
   <223> Description of Artificial Sequence: Synthetic primer
<400> 79
   aggacgaata agtgccactc 20
<210> 80
   <211> 39
   <212> DNA
   <213> Artificial Sequence
<220>
   <223> Description of Artificial Sequence: Synthetic primer
<400> 80
   gcctccctcg cgccatcaga ggacgaataa gtgccactc 39
<210> 81
   <211> 20
   <212> DNA
   <213> Artificial Sequence
<220>
   <223> Description of Artificial Sequence: Synthetic primer
<400> 81
   tcaccacgta ctgcaattcc 20
<210> 82
   <211> 20
   <212> DNA
   <213> Artificial Sequence
<220>
   <223> Description of Artificial Sequence: Synthetic primer
<400> 82
   agactgacat ttcagtttgg 20
<210> 83
   <211> 39
   <212> DNA
   <213> Artificial Sequence
<220>
   <223> Description of Artificial Sequence: Synthetic primer
<400> 83
   gcctccctcg cgccatcaga gactgacatt tcagtttgg 39
<210> 84
   <211> 20
   <212> DNA
   <213> Artificial Sequence
<220>
   <223> Description of Artificial Sequence: Synthetic primer
<400> 84
   tcgacagatt cmctcccagg 20
<210> 85
   <211> 20
   <212> DNA
   <213> Artificial Sequence
<220>
   <223> Description of Artificial Sequence: Synthetic primer
<400> 85
   gtccagyacc ttgatcctgc 20
<210> 86
   <211> 39
   <212> DNA
   <213> Artificial Sequence
<220>
   <223> Description of Artificial Sequence: Synthetic primer
<400> 86
   gcctccctcg cgccatcagg tccagyacct tgatcctgc 39
<210> 87
   <211> 20
   <212> DNA
   <213> Artificial Sequence
<220>
   <223> Description of Artificial Sequence: Synthetic primer
<400> 87
   cctcaagtgt tttttccagc 20
<210> 88
   <211> 20
   <212> DNA
   <213> Artificial Sequence
<220>
   <223> Description of Artificial Sequence: Synthetic primer
<400> 88
   gtgacagtag ttacgggtgg 20
<210> 89
   <211> 39
   <212> DNA
   <213> Artificial Sequence
<220>
   <223> Description of Artificial Sequence: Synthetic primer
<400> 89
   gcctccctcg cgccatcagg tgacagtagt tacgggtgg 39
<210> 90
   <211> 20
   <212> DNA
   <213> Artificial Sequence
<220>
   <223> Description of Artificial Sequence: Synthetic primer
<400> 90
   cagcatgttt gattatttcc 20
<210> 91
   <211> 20
   <212> DNA
   <213> Artificial Sequence
<220>
   <223> Description of Artificial Sequence: Synthetic primer
<400> 91
   atctataagt tccattaagg 20
<210> 92
   <211> 39
   <212> DNA
   <213> Artificial Sequence
<220>
   <223> Description of Artificial Sequence: Synthetic primer
<400> 92
   gcctccctcg cgccatcaga tctataagtt ccattaagg 39
<210> 93
   <211> 20
   <212> DNA
   <213> Artificial Sequence
<220>
   <223> Description of Artificial Sequence: Synthetic primer
<400> 93
   ctccaaggct ttatattctg 20
<210> 94
   <211> 20
   <212> DNA
   <213> Artificial Sequence
<220>
   <223> Description of Artificial Sequence: Synthetic primer
<400> 94
   atgatattac tgaagggtgg 20
<210> 95
   <211> 39
   <212> DNA
   <213> Artificial Sequence
<220>
   <223> Description of Artificial Sequence: Synthetic primer
<400> 95
   gcctccctcg cgccatcaga tgatattact gaagggtgg 39
<210> 96
   <211> 20
   <212> DNA
   <213> Artificial Sequence
<220>
   <223> Description of Artificial Sequence: Synthetic primer
<400> 96
   actgcacgtc atcaaagacg 20
<210> 97
   <211> 20
   <212> DNA
   <213> Artificial Sequence
<220>
   <223> Description of Artificial Sequence: Synthetic primer
<400> 97
   ttgatgatgc tacagaaagg 20
<210> 98
   <211> 39
   <212> DNA
   <213> Artificial Sequence
<220>
   <223> Description of Artificial Sequence: Synthetic primer
<400> 98
   gcctccctcg cgccatcagt tgatgatgct acagaaagg 39
<210> 99
   <211> 20
   <212> DNA
   <213> Artificial Sequence
<220>
   <223> Description of Artificial Sequence: Synthetic primer
<400> 99
   tgaactgcac ttcttcaagc 20
<210> 100
   <211> 20
   <212> DNA
   <213> Artificial Sequence
<220>
   <223> Description of Artificial Sequence: Synthetic primer
<400> 100
   cttgatagcc ttatataagg 20
<210> 101
   <211> 39
   <212> DNA
   <213> Artificial Sequence
<220>
   <223> Description of Artificial Sequence: Synthetic primer
<400> 101
   gcctccctcg cgccatcagc ttgatagcct tatataagg 39
<210> 102
   <211> 20
   <212> DNA
   <213> Artificial Sequence
<220>
   <223> Description of Artificial Sequence: Synthetic primer
<400> 102
   tcaattgcag ttatgaagtg 20
<210> 103
   <211> 20
   <212> DNA
   <213> Artificial Sequence
<220>
   <223> Description of Artificial Sequence: Synthetic primer
<400> 103
   tttatgctaa cttcaagtgg 20
<210> 104
   <211> 39
   <212> DNA
   <213> Artificial Sequence
<220>
   <223> Description of Artificial Sequence: Synthetic primer
<400> 104
   gcctccctcg cgccatcagt ttatgctaac ttcaagtgg 39
<210> 105
   <211> 20
   <212> DNA
   <213> Artificial Sequence
<220>
   <223> Description of Artificial Sequence: Synthetic primer
<400> 105
   gcacatatga caccagtgag 20
<210> 106
   <211> 20
   <212> DNA
   <213> Artificial Sequence
<220>
   <223> Description of Artificial Sequence: Synthetic primer
<400> 106
   tcgccaagaa gcttataagc 20
<210> 107
   <211> 39
   <212> DNA
   <213> Artificial Sequence
<220>
   <223> Description of Artificial Sequence: Synthetic primer
<400> 107
   gcctccctcg cgccatcagt cgccaagaag cttataagc 39
<210> 108
   <211> 20
   <212> DNA
   <213> Artificial Sequence
<220>
   <223> Description of Artificial Sequence: Synthetic primer
<400> 108
   tctactgcaa ttattcaacc 20
<210> 109
   <211> 20
   <212> DNA
   <213> Artificial Sequence
<220>
   <223> Description of Artificial Sequence: Synthetic primer
<400> 109
   caggagggac gattaatggc 20
<210> 110
   <211> 39
   <212> DNA
   <213> Artificial Sequence
<220>
   <223> Description of Artificial Sequence: Synthetic primer
<400> 110
   gcctccctcg cgccatcagc aggagggacg attaatggc 39
<210> 111
   <211> 20
   <212> DNA
   <213> Artificial Sequence
<220>
   <223> Description of Artificial Sequence: Synthetic primer
<400> 111
   tgaactgcac atacacatcc 20
<210> 112
   <211> 20
   <212> DNA
   <213> Artificial Sequence
<220>
   <223> Description of Artificial Sequence: Synthetic primer
<400> 112
   acagcaaaaa cttcggaggc 20
<210> 113
   <211> 39
   <212> DNA
   <213> Artificial Sequence
<220>
   <223> Description of Artificial Sequence: Synthetic primer
<400> 113
   gcctccctcg cgccatcaga cagcaaaaac ttcggaggc 39
<210> 114
   <211> 20
   <212> DNA
   <213> Artificial Sequence
<220>
   <223> Description of Artificial Sequence: Synthetic primer
<400> 114
   aactgcagtt actcggtagg 20
<210> 115
   <211> 20
   <212> DNA
   <213> Artificial Sequence
<220>
   <223> Description of Artificial Sequence: Synthetic primer
<400> 115
   aagcatggaa gattaattgc 20
<210> 116
   <211> 39
   <212> DNA
   <213> Artificial Sequence
<220>
   <223> Description of Artificial Sequence: Synthetic primer
<400> 116
   gcctccctcg cgccatcaga agcatggaag attaattgc 39
<210> 117
   <211> 20
   <212> DNA
   <213> Artificial Sequence
<220>
   <223> Description of Artificial Sequence: Synthetic primer
<400> 117
   gcagacagac ttgtcactgg 20
<210> 118
   <211> 20
   <212> DNA
   <213> Artificial Sequence
<220>
   <223> Description of Artificial Sequence: Synthetic primer
<400> 118
   agtctctcag ctggtacacg 20
<210> 119
   <211> 39
   <212> DNA
   <213> Artificial Sequence
<220>
   <223> Description of Artificial Sequence: Synthetic primer
<400> 119
   gccttgccag cccgctcaga gtctctcagc tggtacacg 39
<210> 120
   <211> 20
   <212> DNA
   <213> Artificial Sequence
<220>
   <223> Description of Artificial Sequence: Synthetic primer
<400> 120
   aatgaaacgt gagcatctgg 20
<210> 121
   <211> 20
   <212> DNA
   <213> Artificial Sequence
<220>
   <223> Description of Artificial Sequence: Synthetic primer
<400> 121
   cattgaaaac aagactgtgc 20
<210> 122
   <211> 39
   <212> DNA
   <213> Artificial Sequence
<220>
   <223> Description of Artificial Sequence: Synthetic primer
<400> 122
   gcctccctcg cgccatcagc attgaaaaca agactgtgc 39
<210> 123
   <211> 20
   <212> DNA
   <213> Artificial Sequence
<220>
   <223> Description of Artificial Sequence: Synthetic primer
<400> 123
   gtgtccccat ctctaatcac 20
<210> 124
   <211> 20
   <212> DNA
   <213> Artificial Sequence
<220>
   <223> Description of Artificial Sequence: Synthetic primer
<400> 124
   tgaaatctca gagaagtctg 20
<210> 125
   <211> 39
   <212> DNA
   213> Artificial Sequence
<220>
   <223> Description of Artificial Sequence: Synthetic primer
<400> 125
   gcctccctcg cgccatcagt gaaatctcag agaagtctg 39
<210> 126
   <211> 20
   <212> DNA
   <213> Artificial Sequence
<220>
   <223> Description of Artificial Sequence: Synthetic primer
<400> 126
   tatgtattgg tataaacagg 20
<210> 127
   <211> 20
   <212> DNA
   <213> Artificial Sequence
<220>
   <223> Description of Artificial Sequence: Synthetic primer
<400> 127
   ctctaagaaa tttctgaaga 20
<210> 128
   <211> 39
   <212> DNA
   <213> Artificial Sequence
<220>
   <223> Description of Artificial Sequence: Synthetic primer
<400> 128
   gcctccctcg cgccatcagc tctaagaaat ttctgaaga 39
<210> 129
   <211> 20
   <212> DNA
   <213> Artificial Sequence
<220>
   <223> Description of Artificial Sequence: Synthetic primer
<400> 129
   gtctttgaaa tgtgaacaac 20
<210> 130
   <211> 20
   <212> DNA
   <213> Artificial Sequence
<220>
   <223> Description of Artificial Sequence: Synthetic primer
<400> 130
   ggagctcatg tttgtctaca 20
<210> 131
   <211> 39
   <212> DNA
   <213> Artificial Sequence
<220>
   <223> Description of Artificial Sequence: Synthetic primer
<400> 131
   gcctccctcg cgccatcagg gagctcatgt ttgtctaca 39
<210> 132
   <211> 20
   <212> DNA
   <213> Artificial Sequence
<220>
   <223> Description of Artificial Sequence: Synthetic primer
<400> 132
   gatcaaaacg agaggacagc 20
<210> 133
   <211> 20
   <212> DNA
   <213> Artificial Sequence
<220>
   <223> Description of Artificial Sequence: Synthetic primer
<400> 133
   caggggcccc agtttatctt 20
<210> 134
   <211> 39
   <212> DNA
   <213> Artificial Sequence
<220>
   <223> Description of Artificial Sequence: Synthetic primer
<400> 134
   gcctccctcg cgccatcagc aggggcccca gtttatctt 39
<210> 135
   <211> 20
   <212> DNA
   <213> Artificial Sequence
<220>
   <223> Description of Artificial Sequence: Synthetic primer
<400> 135
   gaaacaragg aaacttccct 20
<210> 136
   <211> 39
   <212> DNA
   <213> Artificial Sequence
<220>
   <223> Description of Artificial Sequence: Synthetic primer
<400> 136
   gcctccctcg cgccatcagg aaacaragga aacttccct 39
<210> 137
   <211> 20
   <212> DNA
   <213> Artificial Sequence
<220>
   <223> Description of Artificial Sequence: Synthetic primer
<400> 137
   gtgtgcccag gatatgaacc 20
<210> 138
   <211> 20
   <212> DNA
   <213> Artificial Sequence
<220>
   <223> Description of Artificial Sequence: Synthetic primer
<400> 138
   caggatatga gacataatgc 20
<210> 139
   <211> 20
   <212> DNA
   <213> Artificial Sequence
<220>
   <223> Description of Artificial Sequence: Synthetic primer
<400> 139
   ggtatcgaca agacccaggc 20
<210> 140
   <211> 39
   <212> DNA
   <213> Artificial Sequence
<220>
   <223> Description of Artificial Sequence: Synthetic primer
<400> 140
   gcctccctcg cgccatcagg gtatcgacaa gacccaggc 39
<210> 141
   <211> 20
   <212> DNA
   <213> Artificial Sequence
<220>
   <223> Description of Artificial Sequence: Synthetic primer
<400> 141
   tagacaagat ctaggactgg 20
<210> 142
   <211> 39
   <212> DNA
   <213> Artificial Sequence
<220>
   <223> Description of Artificial Sequence: Synthetic primer
<400> 142
   gcctccctcg cgccatcagt agacaagatc taggactgg 39
<210> 143
   <211> 20
   <212> DNA
   <213> Artificial Sequence
<220>
   <223> Description of Artificial Sequence: Synthetic primer
<400> 143
   ctcaggtgtg atccaatttc 20
<210> 144
   <211> 20
   <212> DNA
   <213> Artificial Sequence
<220>
   <223> Description of Artificial Sequence: Synthetic primer
<400> 144
   tctaatttac ttccaaggca 20
<210> 145
   <211> 39
   <212> DNA
   <213> Artificial Sequence
<220>
   <223> Description of Artificial Sequence: Synthetic primer
<400> 145
   gcctccctcg cgccatcagt ctaatttact tccaaggca 39
<210> 146
   <211> 20
   <212> DNA
   <213> Artificial Sequence
<220>
   <223> Description of Artificial Sequence: Synthetic primer
<400> 146
   tcccagagtg atgctcaacg 20
<210> 147
   <211> 39
   <212> DNA
   <213> Artificial Sequence
<220>
   <223> Description of Artificial Sequence: Synthetic primer
<400> 147
   gcctccctcg cgccatcagt cccagagtga tgctcaacg 39
<210> 148
   <211> 20
   <212> DNA
   <213> Artificial Sequence
<220>
   <223> Description of Artificial Sequence: Synthetic primer
<400> 148
   acttacttca attatgaagc 20
<210> 149
   <211> 39
   <212> DNA
   <213> Artificial Sequence
<220>
   <223> Description of Artificial Sequence: Synthetic primer
<400> 149
   gcctccctcg cgccatcaga cttacttcaa ttatgaagc 39
<210> 150
   <211> 20
   <212> DNA
   <213> Artificial Sequence
<220>
   <223> Description of Artificial Sequence: Synthetic primer
<400> 150
   ccagaatgaa gctcaactag 20
<210> 151
   <211> 39
   <212> DNA
   <213> Artificial Sequence
<220>
   <223> Description of Artificial Sequence: Synthetic primer
<400> 151
   gcctccctcg cgccatcagc cagaatgaag ctcaactag 39
<210> 152
   <211> 20
   <212> DNA
   <213> Artificial Sequence
<220>
   <223> Description of Artificial Sequence: Synthetic primer
<400> 152
   gagacctctc tgtgtactgg 20
<210> 153
   <211> 20
   <212> DNA
   <213> Artificial Sequence
<220>
   <223> Description of Artificial Sequence: Synthetic primer
<400> 153
   ctcattcagt attataatgg 20
<210> 154
   <211> 39
   <212> DNA
   <213> Artificial Sequence
<220>
   <223> Description of Artificial Sequence: Synthetic primer
<400> 154
   gcctccctcg cgccatcagc tcattcagta ttataatgg 39
<210> 155
   <211> 20
   <212> DNA
   <213> Artificial Sequence
<220>
   <223> Description of Artificial Sequence: Synthetic primer
<400> 155
   ggaatcaccc agagcccaag 20
<210> 156
   <211> 20
   <212> DNA
   <213> Artificial Sequence
<220>
   <223> Description of Artificial Sequence: Synthetic primer
<400> 156
   gacatgggct gaggctgatc 20
<210> 157
   <211> 39
   <212> DNA
   <213> Artificial Sequence
<220>
   <223> Description of Artificial Sequence: Synthetic primer
<400> 157
   gcctccctcg cgccatcagg acatgggctg aggctgatc 39
<210> 158
   <211> 20
   <212> DNA
   <213> Artificial Sequence
<220>
   <223> Description of Artificial Sequence: Synthetic primer
<400> 158
   cctaaggatc gattttctgc 20
<210> 159
   <211> 20
   <212> DNA
   <213> Artificial Sequence
<220>
   <223> Description of Artificial Sequence: Synthetic primer
<400> 159
   actctcaaga tccagcctgc 20
<210> 160
   <211> 39
   <212> DNA
   <213> Artificial Sequence
<220>
   <223> Description of Artificial Sequence: Synthetic primer
<400> 160
   gcctccctcg cgccatcaga ctctcaagat ccagcctgc 39
<210> 161
   <211> 20
   <212> DNA
   <213> Artificial Sequence
<220>
   <223> Description of Artificial Sequence: Synthetic primer
<400> 161
   aggtgacaga gatgggacaa 20
<210> 162
   <211> 20
   <212> DNA
   <213> Artificial Sequence
<220>
   <223> Description of Artificial Sequence: Synthetic primer
<400> 162
   tgcagggact ggaattgctg 20
<210> 163
   <211> 39
   <212> DNA
   <213> Artificial Sequence
<220>
   <223> Description of Artificial Sequence: Synthetic primer
<400> 163
   gcctccctcg cgccatcagt gcagggactg gaattgctg 39
<210> 164
   <211> 20
   <212> DNA
   <213> Artificial Sequence
<220>
   <223> Description of Artificial Sequence: Synthetic primer
<400> 164
   gtacagacag accatgatgc 20
<210> 165
   <211> 39
   <212> DNA
   <213> Artificial Sequence
<220>
   <223> Description of Artificial Sequence: Synthetic primer
<400> 165
   gcctccctcg cgccatcagg tacagacaga ccatgatgc 39
<210> 166
   <211> 20
   <212> DNA
   <213> Artificial Sequence
<220>
   <223> Description of Artificial Sequence: Synthetic primer
<400> 166
   ctatcctatc cctagacacg 20
<210> 167
   <211> 20
   <212> DNA
   <213> Artificial Sequence
<220>
   <223> Description of Artificial Sequence: Synthetic primer
<400> 167
   aagatgcaga gcgataaagg 20
<210> 168
   <211> 39
   <212> DNA
   <213> Artificial Sequence
<220>
   <223> Description of Artificial Sequence: Synthetic primer
<400> 168
   gcctccctcg cgccatcaga agatgcagag cgataaagg 39
<210> 169
   <211> 20
   <212> DNA
   <213> Artificial Sequence
<220>
   <223> Description of Artificial Sequence: Synthetic primer
<400> 169
   agatgtgacc caatttctgg 20
<210> 170
   <211> 20
   <212> DNA
   <213> Artificial Sequence
<220>
   <223> Description of Artificial Sequence: Synthetic primer
<400> 170
   agtctaaaca ggatgagtcc 20
<210> 171
   <211> 39
   <212> DNA
   <213> Artificial Sequence
<220>
   <223> Description of Artificial Sequence: Synthetic primer
<400> 171
   gcctccctcg cgccatcaga gtctaaacag gatgagtcc 39
<210> 172
   <211> 20
   <212> DNA
   <213> Artificial Sequence
<220>
   <223> Description of Artificial Sequence: Synthetic primer
<400> 172
   tcagactttg aaccataacg 20
<210> 173
   <211> 20
   <212> DNA
   <213> Artificial Sequence
<220>
   <223> Description of Artificial Sequence: Synthetic primer
<400> 173
   aaagatttta acaatgaagc 20
<210> 174
   <211> 39
   <212> DNA
   <213> Artificial Sequence
<220>
   <223> Description of Artificial Sequence: Synthetic primer
<400> 174
   gcctccctcg cgccatcaga aagattttaa caatgaagc 39
<210> 175
   <211> 20
   <212> DNA
   <213> Artificial Sequence
<220>
   <223> Description of Artificial Sequence: Synthetic primer
<400> 175
   tattgtgccc caataaaagg 20
<210> 176
   <211> 20
   <212> DNA
   <213> Artificial Sequence
<220>
   <223> Description of Artificial Sequence: Synthetic primer
<400> 176
   aatgtctttg atgaaacagg 20
<210> 177
   <211> 39
   <212> DNA
   <213> Artificial Sequence
<220>
   <223> Description of Artificial Sequence: Synthetic primer
<400> 177
   gcctccctcg cgccatcaga atgtctttga tgaaacagg 39
<210> 178
   <211> 20
   <212> DNA
   <213> Artificial Sequence
<220>
   <223> Description of Artificial Sequence: Synthetic primer
<400> 178
   atccatcttc tggtcacatg 20
<210> 179
   <211> 20
   <212> DNA
   <213> Artificial Sequence
<220>
   <223> Description of Artificial Sequence: Synthetic primer
<400> 179
   aacattgcag ttgattcagg 20
<210> 180
   <211> 39
   <212> DNA
   <213> Artificial Sequence
<220>
   <223> Description of Artificial Sequence: Synthetic primer
<400> 180
   gcctccctcg cgccatcaga acattgcagt tgattcagg 39
<210> 181
   <211> 20
   <212> DNA
   <213> Artificial Sequence
<220>
   <223> Description of Artificial Sequence: Synthetic primer
<400> 181
   gcagcccaat gaaaggacac 20
<210> 182
   <211> 20
   <212> DNA
   <213> Artificial Sequence
<220>
   <223> Description of Artificial Sequence: Synthetic primer
<400> 182
   aatatcatag atgagtcagg 20
<210> 183
   <211> 39
   <212> DNA
   <213> Artificial Sequence
<220>
   <223> Description of Artificial Sequence: Synthetic primer
<400> 183
   gcctccctcg cgccatcaga atatcataga tgagtcagg 39
<210> 184
   <211> 20
   <212> DNA
   <213> Artificial Sequence
<220>
   <223> Description of Artificial Sequence: Synthetic primer
<400> 184
   tgaacagaat ttgaaccacg 20
<210> 185
   <211> 20
   <212> DNA
   <213> Artificial Sequence
<220>
   <223> Description of Artificial Sequence: Synthetic primer
<400> 185
   tttcagaaag gagatatagc 20
<210> 186
   <211> 39
   <212> DNA
   <213> Artificial Sequence
<220>
   <223> Description of Artificial Sequence: Synthetic primer
<400> 186
   gcctccctcg cgccatcagt ttcagaaagg agatatagc 39
<210> 187
   <211> 20
   <212> DNA
   <213> Artificial Sequence
<220>
   <223> Description of Artificial Sequence: Synthetic primer
<400> 187
   tcgagtgccg ttccctggac 20
<210> 188
   <211> 20
   <212> DNA
   <213> Artificial Sequence
<220>
   <223> Description of Artificial Sequence: Synthetic primer
<400> 188
   gatggcaact tccaatgagg 20
<210> 189
   <211> 39
   <212> DNA
   <213> Artificial Sequence
<220>
   <223> Description of Artificial Sequence: Synthetic primer
<400> 189
   gcctccctcg cgccatcagg atggcaactt ccaatgagg 39
<210> 190
   <211> 20
   <212> DNA
   <213> Artificial Sequence
<220>
   <223> Description of Artificial Sequence: Synthetic primer
<400> 190
   gcaaagatgg attgtgttcc 20
<210> 191
   <211> 20
   <212> DNA
   <213> Artificial Sequence
<220>
   <223> Description of Artificial Sequence: Synthetic primer
<400> 191
   cgctggaaga agagctcaag 20
<210> 192
   <211> 39
   <212> DNA
   <213> Artificial Sequence
<220>
   <223> Description of Artificial Sequence: Synthetic primer
<400> 192
   gcctccctcg cgccatcagc gctggaagaa gagctcaag 39
<210> 193
   <211> 20
   <212> DNA
   <213> Artificial Sequence
<220>
   <223> Description of Artificial Sequence: Synthetic primer
<400> 193
   catttggtca aaggaaaagg 20
<210> 194
   <211> 20
   <212> DNA
   <213> Artificial Sequence
<220>
   <223> Description of Artificial Sequence: Synthetic primer
<400> 194
   gaatgaacaa gttcttcaag 20
<210> 195
   <211> 39
   <212> DNA
   <213> Artificial Sequence
<220>
   <223> Description of Artificial Sequence: Synthetic primer
<400> 195
   gcctccctcg cgccatcagg aatgaacaag ttcttcaag 39
<210> 196
   <211> 20
   <212> DNA
   <213> Artificial Sequence
<220>
   <223> Description of Artificial Sequence: Synthetic primer
<400> 196
   atgctggaat gttctcagac 20
<210> 197
   <211> 20
   <212> DNA
   <213> Artificial Sequence
<220>
   <223> Description of Artificial Sequence: Synthetic primer
<400> 197
   gtcaaagata taaacaaagg 20
<210> 198
   <211> 39
   <212> DNA
   <213> Artificial Sequence
<220>
   <223> Description of Artificial Sequence: Synthetic primer
<400> 198
   gcctccctcg cgccatcagg tcaaagatat aaacaaagg 39
<210> 199
   <211> 20
   <212> DNA
   <213> Artificial Sequence
<220>
   <223> Description of Artificial Sequence: Synthetic primer
<400> 199
   ctctggaatg ttctcaaacc 20
<210> 200
   <211> 20
   <212> DNA
   <213> Artificial Sequence
<220>
   <223> Description of Artificial Sequence: Synthetic primer
<400> 200
   taattccaca gagaagggag 20
<210> 201
   <211> 39
   <212> DNA
   <213> Artificial Sequence
<220>
   <223> Description of Artificial Sequence: Synthetic primer
<400> 201
   gcctccctcg cgccatcagt aattccacag agaagggag 39
<210> 202
   <211> 20
   <212> DNA
   <213> Artificial Sequence
<220>
   <223> Description of Artificial Sequence: Synthetic primer
<400> 202
   cccagaatat gaatcatgtt 20
<210> 203
   <211> 20
   <212> DNA
   <213> Artificial Sequence
<220>
   <223> Description of Artificial Sequence: Synthetic primer
<400> 203
   attcacctgg cactgggagc 20
<210> 204
   <211> 39
   <212> DNA
   <213> Artificial Sequence
<220>
   <223> Description of Artificial Sequence: Synthetic primer
<400> 204
   gcctccctcg cgccatcaga ttcacctggc actgggagc 39
<210> 205
   <211> 20
   <212> DNA
   <213> Artificial Sequence
<220>
   <223> Description of Artificial Sequence: Synthetic primer
<400> 205
   ttgttctcag aatatgaacc 20
<210> 206
   <211> 20
   <212> DNA
   <213> Artificial Sequence
<220>
   <223> Description of Artificial Sequence: Synthetic primer
<400> 206
   tgaggtgact gataagggag 20
<210> 207
   <211> 39
   <212> DNA
   <213> Artificial Sequence
<220>
   <223> Description of Artificial Sequence: Synthetic primer
<400> 207
   gcctccctcg cgccatcagt gaggtgactg ataagggag 39
<210> 208
   <211> 20
   <212> DNA
   <213> Artificial Sequence
<220>
   <223> Description of Artificial Sequence: Synthetic primer
<400> 208
   atgtgtccag gatatggacc 20
<210> 209
   <211> 20
   <212> DNA
   <213> Artificial Sequence
<220>
   <223> Description of Artificial Sequence: Synthetic primer
<400> 209
   aaaaggagat attcctgagg 20
<210> 210
   <211> 39
   <212> DNA
   <213> Artificial Sequence
<220>
   <223> Description of Artificial Sequence: Synthetic primer
<400> 210
   gcctccctcg cgccatcaga aaaggagata ttcctgagg 39
<210> 211
   <211> 20
   <212> DNA
   <213> Artificial Sequence
<220>
   <223> Description of Artificial Sequence: Synthetic primer
<400> 211
   tcaccatgat gttctggtac 20
<210> 212
   <211> 20
   <212> DNA
   <213> Artificial Sequence
<220>
   <223> Description of Artificial Sequence: Synthetic primer
<400> 212
   ctggacagag cctgacactg 20
<210> 213
   <211> 39
   <212> DNA
   <213> Artificial Sequence
<220>
   <223> Description of Artificial Sequence: Synthetic primer
<400> 213
   gcctccctcg cgccatcagc tggacagagc ctgacactg 39
<210> 214
   <211> 20
   <212> DNA
   <213> Artificial Sequence
<220>
   <223> Description of Artificial Sequence: Synthetic primer
<400> 214
   tgtggaggga acatcaaacc 20
<210> 215
   <211> 20
   <212> DNA
   <213> Artificial Sequence
<220>
   <223> Description of Artificial Sequence: Synthetic primer
<400> 215
   ttctactccg ttggtattgg 20
<210> 216
   <211> 39
   <212> DNA
   <213> Artificial Sequence
<220>
   <223> Description of Artificial Sequence: Synthetic primer
<400> 216
   gcctccctcg cgccatcagt tctactccgt tggtattgg 39
<210> 217
   <211> 20
   <212> DNA
   <213> Artificial Sequence
<220>
   <223> Description of Artificial Sequence: Synthetic primer
<400> 217
   gtgtggcctt ttgggtgtgg 20
<210> 218
   <211> 20
   <212> DNA
   <213> Artificial Sequence
<220>
   <223> Description of Artificial Sequence: Synthetic primer
<400> 218
   tctgatggct caaacacagc 20
<210> 219
   <211> 39
   <212> DNA
   <213> Artificial Sequence
<220>
   <223> Description of Artificial Sequence: Synthetic primer
<400> 219
   gccttgccag cccgctcagt ctgatggctc aaacacagc 39
<210> 220
   <211> 20
   <212> DNA
   <213> Artificial Sequence
<220>
   <223> Description of Artificial Sequence: Synthetic primer
<400> 220
   tgtatgaaac aagttggtgg 20
<210> 221
   <211> 20
   <212> DNA
   <213> Artificial Sequence
<220>
   <223> Description of Artificial Sequence: Synthetic primer
<400> 221
   cagaatgcaa aaagtggtcg 20
<210> 222
   <211> 39
   <212> DNA
   <213> Artificial Sequence
<220>
   <223> Description of Artificial Sequence: Synthetic primer
<400> 222
   gcctccctcg cgccatcagc agaatgcaaa aagtggtcg 39
<210> 223
   <211> 20
   <212> DNA
   <213> Artificial Sequence
<220>
   <223> Description of Artificial Sequence: Synthetic primer
<400> 223
   atgaaaggag aagcgatcgg 20
<210> 224
   <211> 20
   <212> DNA
   <213> Artificial Sequence
<220>
   <223> Description of Artificial Sequence: Synthetic primer
<400> 224
   tggtttcaaa gacaatttcc 20
<210> 225
   <211> 39
   <212> DNA
   <213> Artificial Sequence
<220>
   <223> Description of Artificial Sequence: Synthetic primer
<400> 225
   gcctccctcg cgccatcagt ggtttcaaag acaatttcc 39
<210> 226
   <211> 20
   <212> DNA
   <213> Artificial Sequence
<220>
   <223> Description of Artificial Sequence: Synthetic primer
<400> 226
   gacactgtat attcaaatcc 20
<210> 227
   <211> 20
   <212> DNA
   <213> Artificial Sequence
<220>
   <223> Description of Artificial Sequence: Synthetic primer
<400> 227
   gcagatttta ctcaaggacg 20
<210> 228
   <211> 39
   <212> DNA
   <213> Artificial Sequence
<220>
   <223> Description of Artificial Sequence: Synthetic primer
<400> 228
   gcctccctcg cgccatcagg cagattttac tcaaggacg 39
<210> 229
   <211> 20
   <212> DNA
   <213> Artificial Sequence
<220>
   <223> Description of Artificial Sequence: Synthetic primer
<400> 229
   agacaagcga catttgttcc 20
<210> 230
   <211> 20
   <212> DNA
   <213> Artificial Sequence
<220>
   <223> Description of Artificial Sequence: Synthetic primer
<400> 230
   acggatggtt tggtatgagg 20
<210> 231
   <211> 39
   <212> DNA
   <213> Artificial Sequence
<220>
   <223> Description of Artificial Sequence: Synthetic primer
<400> 231
   gccttgccag cccgctcaga cggatggttt ggtatgagg 39
<210> 232
   <211> 20
   <212> DNA
   <213> Artificial Sequence
<220>
   <223> Description of Artificial Sequence: Synthetic primer
<400> 232
   gggtcatctg ctgaaatcac 20
<210> 233
   <211> 20
   <212> DNA
   <213> Artificial Sequence
<220>
   <223> Description of Artificial Sequence: Synthetic primer
<400> 233
   aggaggggaa ggccccacag 20
<210> 234
   <211> 39
   <212> DNA
   <213> Artificial Sequence
<220>
   <223> Description of Artificial Sequence: Synthetic primer
<400> 234
   gcctccctcg cgccatcaga ggaggggaag gccccacag 39
<210> 235
   <211> 20
   <212> DNA
   <213> Artificial Sequence
<220>
   <223> Description of Artificial Sequence: Synthetic primer
<400> 235
   gggtcatcag ctgtaatcac 20
<210> 236
   <211> 20
   <212> DNA
   <213> Artificial Sequence
<220>
   <223> Description of Artificial Sequence: Synthetic primer
<400> 236
   aggaggggaa gaccccacag 20
<210> 237
   <211> 39
   <212> DNA
   <213> Artificial Sequence
<220>
   <223> Description of Artificial Sequence: Synthetic primer
<400> 237
   gcctccctcg cgccatcaga ggaggggaag accccacag 39
<210> 238
   <211> 20
   <212> DNA
   <213> Artificial Sequence
<220>
   <223> Description of Artificial Sequence: Synthetic primer
<400> 238
   agcccgcctg gaatgtgtgg 20
<210> 239
   <211> 20
   <212> DNA
   <213> Artificial Sequence
<220>
   <223> Description of Artificial Sequence: Synthetic primer
<400> 239
   gcactgtcag aaaggaatcc 20
<210> 240
   <211> 39
   <212> DNA
   <213> Artificial Sequence
<220>
   <223> Description of Artificial Sequence: Synthetic primer
<400> 240
   gcctccctcg cgccatcagg cactgtcaga aaggaatcc 39
<210> 241
   <211> 20
   <212> DNA
   <213> Artificial Sequence
<220>
   <223> Description of Artificial Sequence: Synthetic primer
<400> 241
   aagaaaagta ttgacatacc 20
<210> 242
   <211> 20
   <212> DNA
   <213> Artificial Sequence
<220>
   <223> Description of Artificial Sequence: Synthetic primer
<400> 242
   atattgtctc aacaaaatcc 20
<210> 243
   <211> 39
   <212> DNA
   <213> Artificial Sequence
<220>
   <223> Description of Artificial Sequence: Synthetic primer
<400> 243
   gcctccctcg cgccatcaga tattgtctca acaaaatcc 39
<210> 244
   <211> 20
   <212> DNA
   <213> Artificial Sequence
<220>
   <223> Description of Artificial Sequence: Synthetic primer
<400> 244
   agagtgccca catatcttgg 20
<210> 245
   <211> 20
   <212> DNA
   <213> Artificial Sequence
<220>
   <223> Description of Artificial Sequence: Synthetic primer
<400> 245
   gctcaagatt gctcaggtgg 20
<210> 246
   <211> 39
   <212> DNA
   <213> Artificial Sequence
<220>
   <223> Description of Artificial Sequence: Synthetic primer
<400> 246
   gcctccctcg cgccatcagg ctcaagattg ctcaggtgg 39
<210> 247
   <211> 20
   <212> DNA
   <213> Artificial Sequence
<220>
   <223> Description of Artificial Sequence: Synthetic primer
<400> 247
   ggatcccaga atcgtgttgc 20
<210> 248
   <211> 20
   <212> DNA
   <213> Artificial Sequence
<220>
   <223> Description of Artificial Sequence: Synthetic primer
<400> 248
   ggtatgttcc agccttctgg 20
<210> 249
   <211> 39
   <212> DNA
   <213> Artificial Sequence
<220>
   <223> Description of Artificial Sequence: Synthetic primer
<400> 249
   gccttgccag cccgctcagg gtatgttcca gccttctgg 39
<210> 250
   <211> 20
   <212> DNA
   <213> Artificial Sequence
<220>
   <223> Description of Artificial Sequence: Synthetic primer
<400> 250
   agtgaaggtc tcctgcaagg 20
<210> 251
   <211> 20
   <212> DNA
   <213> Artificial Sequence
<220>
   <223> Description of Artificial Sequence: Synthetic primer
<400> 251
   agtgaaggtt tcctgcaagg 20
<210> 252
   <211> 19
   <212> DNA
   <213> Artificial Sequence
<220>
   <223> Description of Artificial Sequence: Synthetic primer
<400> 252
   agttccaggg cagagtcac 19
<210> 253
   <211> 38
   <212> DNA
   <213> Artificial Sequence
<220>
   <223> Description of Artificial Sequence: Synthetic primer
<400> 253
   gcctccctcg cgccatcaga gttccagggc agagtcac 38
<210> 254
   <211> 19
   <212> DNA
   <213> Artificial Sequence
<220>
   <223> Description of Artificial Sequence: Synthetic primer
<400> 254
   agtttcaggg cagggtcac 19
<210> 255
   <211> 38
   <212> DNA
   <213> Artificial Sequence
<220>
   <223> Description of Artificial Sequence: Synthetic primer
<400> 255
   gcctccctcg cgccatcaga gtttcagggc agggtcac 38
<210> 256
   <211> 19
   <212> DNA
   <213> Artificial Sequence
<220>
   <223> Description of Artificial Sequence: Synthetic primer
<400> 256
   agttccagga aagagtcac 19
<210> 257
   <211> 38
   <212> DNA
   <213> Artificial Sequence
<220>
   <223> Description of Artificial Sequence: Synthetic primer
<400> 257
   gcctccctcg cgccatcaga gttccaggaa agagtcac 38
<210> 258
   <211> 19
   <212> DNA
   <213> Artificial Sequence
<220>
   <223> Description of Artificial Sequence: Synthetic primer
<400> 258
   aattccagga cagagtcac 19
<210> 259
   <211> 38
   <212> DNA
   <213> Artificial Sequence
<220>
   <223> Description of Artificial Sequence: Synthetic primer
<400> 259
   gcctccctcg cgccatcaga attccaggac agagtcac 38
<210> 260
   <211> 20
   <212> DNA
   <213> Artificial Sequence
<220>
   <223> Description of Artificial Sequence: Synthetic primer
<400> 260
   tctctgggtt ctcactcagc 20
<210> 261
   <211> 20
   <212> DNA
   <213> Artificial Sequence
<220>
   <223> Description of Artificial Sequence: Synthetic primer
<400> 261
   aaggccctgg agtggcttgc 20
<210> 262
   <211> 39
   <212> DNA
   <213> Artificial Sequence
<220>
   <223> Description of Artificial Sequence: Synthetic primer
<400> 262
   gcctccctcg cgccatcaga aggccctgga gtggcttgc 39
<210> 263
   <211> 20
   <212> DNA
   <213> Artificial Sequence
<220>
   <223> Description of Artificial Sequence: Synthetic primer
<400> 263
   tccctgagac tctcctgtgc 20
<210> 264
   <211> 20
   <212> DNA
   <213> Artificial Sequence
<220>
   <223> Description of Artificial Sequence: Synthetic primer
<400> 264
   ctctcctgtg cagcctctgg 20
<210> 265
   <211> 20
   <212> DNA
   <213> Artificial Sequence
<220>
   <223> Description of Artificial Sequence: Synthetic primer
<400> 265
   ggtccctgag actctcctgt 20
<210> 266
   <211> 20
   <212> DNA
   <213> Artificial Sequence
<220>
   <223> Description of Artificial Sequence: Synthetic primer
<400> 266
   ctgagactct cctgtgtagc 20
<210> 267
   <211> 18
   <212> DNA
   <213> Artificial Sequence
<220>
   <223> Description of Artificial Sequence: Synthetic primer
<400> 267
   ctccagggaa ggggctgg 18
<210> 268
   <211> 37
   <212> DNA
   <213> Artificial Sequence
<220>
   <223> Description of Artificial Sequence: Synthetic primer
<400> 268
   gcctccctcg cgccatcagc tccagggaag gggctgg 37
<210> 269
   <211> 18
   <212> DNA
   <213> Artificial Sequence
<220>
   <223> Description of Artificial Sequence: Synthetic primer
<400> 269
   ggctccaggc aaggggct 18
<210> 270
   <211> 37
   <212> DNA
   <213> Artificial Sequence
<220>
   <223> Description of Artificial Sequence: Synthetic primer
<400> 270
   gcctccctcg cgccatcagg gctccaggca aggggct 37
<210> 271
   <211> 19
   <212> DNA
   <213> Artificial Sequence
<220>
   <223> Description of Artificial Sequence: Synthetic primer
<400> 271
   actgggtccg ccaggctcc 19
<210> 272
   <211> 38
   <212> DNA
   <213> Artificial Sequence
<220>
   <223> Description of Artificial Sequence: Synthetic primer
<400> 272
   gcctccctcg cgccatcaga ctgggtccgc caggctcc 38
<210> 273
   <211> 18
   <212> DNA
   <213> Artificial Sequence
<220>
   <223> Description of Artificial Sequence: Synthetic primer
<400> 273
   gaaggggctg gagtgggt 18
<210> 274
   <211> 37
   <212> DNA
   <213> Artificial Sequence
<220>
   <223> Description of Artificial Sequence: Synthetic primer
<400> 274
   gcctccctcg cgccatcagg aaggggctgg agtgggt 37
<210> 275
   <211> 18
   <212> DNA
   <213> Artificial Sequence
<220>
   <223> Description of Artificial Sequence: Synthetic primer
<400> 275
   aaaaggtctg gagtgggt 18
<210> 276
   <211> 37
   <212> DNA
   <213> Artificial Sequence
<220>
   <223> Description of Artificial Sequence: Synthetic primer
<400> 276
   gcctccctcg cgccatcaga aaaggtctgg agtgggt 37
<210> 277
   <211> 20
   <212> DNA
   <213> Artificial Sequence
<220>
   <223> Description of Artificial Sequence: Synthetic primer
<400> 277
   agaccctgtc cctcacctgc 20
<210> 278
   <211> 20
   <212> DNA
   <213> Artificial Sequence
<220>
   <223> Description of Artificial Sequence: Synthetic primer
<400> 278
   agggvctgga gtggattggg 20
<210> 279
   <211> 39
   <212> DNA
   <213> Artificial Sequence
<220>
   <223> Description of Artificial Sequence: Synthetic primer
<400> 279
   gcctccctcg cgccatcaga gggvctggag tggattggg 39
<210> 280
   <211> 20
   <212> DNA
   <213> Artificial Sequence
<220>
   <223> Description of Artificial Sequence: Synthetic primer
<400> 280
   gcgccagatg cccgggaaag 20
<210> 281
   <211> 20
   <212> DNA
   <213> Artificial Sequence
<220>
   <223> Description of Artificial Sequence: Synthetic primer
<400> 281
   ggccasgtca ccatctcagc 20
<210> 282
   <211> 39
   <212> DNA
   <213> Artificial Sequence
<220>
   <223> Description of Artificial Sequence: Synthetic primer
<400> 282
   gcctccctcg cgccatcagg gccasgtcac catctcagc 39
<210> 283
   <211> 20
   <212> DNA
   <213> Artificial Sequence
<220>
   <223> Description of Artificial Sequence: Synthetic primer
<400> 283
   ccggggacag tgtctctagc 20
<210> 284
   <211> 20
   <212> DNA
   <213> Artificial Sequence
<220>
   <223> Description of Artificial Sequence: Synthetic primer
<400> 284
   gccttgagtg gctgggaagg 20
<210> 285
   <211> 39
   <212> DNA
   <213> Artificial Sequence
<220>
   <223> Description of Artificial Sequence: Synthetic primer
<400> 285
   gcctccctcg cgccatcagg ccttgagtgg ctgggaagg 39
<210> 286
   <211> 20
   <212> DNA
   <213> Artificial Sequence
<220>
   <223> Description of Artificial Sequence: Synthetic primer
<400> 286
   gtttcctgca aggcttctgg 20
<210> 287
   <211> 20
   <212> DNA
   <213> Artificial Sequence
<220>
   <223> Description of Artificial Sequence: Synthetic primer
<400> 287
   ggcttgagtg gatgggatgg 20
<210> 288
   <211> 39
   <212> DNA
   <213> Artificial Sequence
<220>
   <223> Description of Artificial Sequence: Synthetic primer
<400> 288
   gcctccctcg cgccatcagg gcttgagtgg atgggatgg 39
<210> 289
   <211> 19
   <212> DNA
   <213> Artificial Sequence
<220>
   <223> Description of Artificial Sequence: Synthetic primer
<400> 289
   acctgaggag acggtgacc 19
<210> 290
   <211> 20
   <212> DNA
   <213> Artificial Sequence
<220>
   <223> Description of Artificial Sequence: Synthetic primer
<400> 290
   cagtgctgga agtattcagc 20
<210> 291
   <211> 39
   <212> DNA
   <213> Artificial Sequence
<220>
   <223> Description of Artificial Sequence: Synthetic primer
<400> 291
   gccttgccag cccgctcagc agtgctggaa gtattcagc 39
<210> 292
   <211> 20
   <212> DNA
   <213> Artificial Sequence
<220>
   <223> Description of Artificial Sequence: Synthetic primer
<400> 292
   agagatcgaa gtaccagtag 20
<210> 293
   <211> 39
   <212> DNA
   <213> Artificial Sequence
<220>
   <223> Description of Artificial Sequence: Synthetic primer
<400> 293
   gccttgccag cccgctcaga gagatcgaag taccagtag 39
<210> 294
   <211> 20
   <212> DNA
   <213> Artificial Sequence
<220>
   <223> Description of Artificial Sequence: Synthetic primer
<400> 294
   ccccagatat caaaagcatc 20
<210> 295
   <211> 39
   <212> DNA
   <213> Artificial Sequence
<220>
   <223> Description of Artificial Sequence: Synthetic primer
<400> 295
   gccttgccag cccgctcagc cccagatatc aaaagcatc 39
<210> 296
   <211> 20
   <212> DNA
   <213> Artificial Sequence
<220>
   <223> Description of Artificial Sequence: Synthetic primer
<400> 296
   ggccccagta gtcaaagtag 20
<210> 297
   <211> 39
   <212> DNA
   <213> Artificial Sequence
<220>
   <223> Description of Artificial Sequence: Synthetic primer
<400> 297
   gccttgccag cccgctcagg gccccagtag tcaaagtag 39
<210> 298
   <211> 20
   <212> DNA
   <213> Artificial Sequence
<220>
   <223> Description of Artificial Sequence: Synthetic primer
<400> 298
   cccaggggtc gaaccagttg 20
<210> 299
   <211> 39
   <212> DNA
   <213> Artificial Sequence
<220>
   <223> Description of Artificial Sequence: Synthetic primer
<400> 299
   gccttgccag cccgctcagc ccaggggtcg aaccagttg 39
<210> 300
   <211> 20
   <212> DNA
   <213> Artificial Sequence
<220>
   <223> Description of Artificial Sequence: Synthetic primer
<400> 300
   cccagacgtc catgtagtag 20
<210> 301
   <211> 39
   <212> DNA
   <213> Artificial Sequence
<220>
   <223> Description of Artificial Sequence: Synthetic primer
<400> 301
   gccttgccag cccgctcagc ccagacgtcc atgtagtag 39
<210> 302
   <211> 20
   <212> DNA
   <213> Artificial Sequence
<220>
   <223> Description of Artificial Sequence: Synthetic primer
<400> 302
   taggagacag agtcaccatc 20
<210> 303
   <211> 20
   <212> DNA
   <213> Artificial Sequence
<220>
   <223> Description of Artificial Sequence: Synthetic primer
<400> 303
   ttcagygrca gtggatctgg 20
<210> 304
   <211> 39
   <212> DNA
   <213> Artificial Sequence
<220>
   <223> Description of Artificial Sequence: Synthetic primer
<400> 304
   gcctccctcg cgccatcagt tcagygrcag tggatctgg 39
<210> 305
   <211> 20
   <212> DNA
   <213> Artificial Sequence
<220>
   <223> Description of Artificial Sequence: Synthetic primer
<400> 305
   ggagagccgg cctccatctc 20
<210> 306
   <211> 20
   <212> DNA
   <213> Artificial Sequence
<220>
   <223> Description of Artificial Sequence: Synthetic primer
<400> 306
   tggtacctgc agaagccagg 20
<210> 307
   <211> 39
   <212> DNA
   <213> Artificial Sequence
<220>
   <223> Description of Artificial Sequence: Synthetic primer
<400> 307
   gcctccctcg cgccatcagt ggtacctgca gaagccagg 39
<210> 308
   <211> 20
   <212> DNA
   <213> Artificial Sequence
<220>
   <223> Description of Artificial Sequence: Synthetic primer
<400> 308
   cttcagcaga ggccaggcca 20
<210> 309
   <211> 39
   <212> DNA
   <213> Artificial Sequence
<220>
   <223> Description of Artificial Sequence: Synthetic primer
<400> 309
   gcctccctcg cgccatcagc ttcagcagag gccaggcca 39
<210> 310
   <211> 20
   <212> DNA
   <213> Artificial Sequence
<220>
   <223> Description of Artificial Sequence: Synthetic primer
<400> 310
   gcctggtacc agcagaaacc 20
<210> 311
   <211> 20
   <212> DNA
   <213> Artificial Sequence
<220>
   <223> Description of Artificial Sequence: Synthetic primer
<400> 311
   gccaggttca gtggcagtgg 20
<210> 312
   <211> 39
   <212> DNA
   <213> Artificial Sequence
<220>
   <223> Description of Artificial Sequence: Synthetic primer
<400> 312
   gcctccctcg cgccatcagg ccaggttcag tggcagtgg 39
<210> 313
   <211> 20
   <212> DNA
   <213> Artificial Sequence
<220>
   <223> Description of Artificial Sequence: Synthetic primer
<400> 313
   tcgaggttca gtggcagtgg **2**0
<210> 314
   <211> 39
   <212> DNA
   <213> Artificial Sequence
<220>
   <223> Description of Artificial Sequence: Synthetic primer
<400> 314
   gcctccctcg cgccatcagt cgaggttcag tggcagtgg 39
<210> 315
   <211> 20
   <212> DNA
   <213> Artificial Sequence
<220>
   <223> Description of Artificial Sequence: Synthetic primer
<400> 315
   gaccgattca gtggcagcgg 20
<210> 316
   <211> 39
   <212> DNA
   <213> Artificial Sequence
<220>
   <223> Description of Artificial Sequence: Synthetic primer
<400> 316
   gcctccctcg cgccatcagg accgattcag tggcagcgg 39
<210> 317
   <211> 20
   <212> DNA
   <213> Artificial Sequence
<220>
   <223> Description of Artificial Sequence: Synthetic primer
<400> 317
   ttcaactgct catcagatgg 20
<210> 318
   <211> 20
   <212> DNA
   <213> Artificial Sequence
<220>
   <223> Description of Artificial Sequence: Synthetic primer
<400> 318
   atgaagacag atggtgcagc 20
<210> 319
   <211> 39
   <212> DNA
   <213> Artificial Sequence
<220>
   <223> Description of Artificial Sequence: Synthetic primer
<400> 319
   gccttgccag cccgctcaga tgaagacaga tggtgcagc 39
<210> 320
   <211> 20
   <212> DNA
   <213> Artificial Sequence
<220>
   <223> Description of Artificial Sequence: Synthetic primer
<400> 320
   gggcagaggg tcaccatctc 20
<210> 321
   <211> 20
   <212> DNA
   <213> Artificial Sequence
<220>
   <223> Description of Artificial Sequence: Synthetic primer
<400> 321
   ggacagaagg tcaccatctc 20
<210> 322
   <211> 20
   <212> DNA
   <213> Artificial Sequence
<220>
   <223> Description of Artificial Sequence: Synthetic primer
<400> 322
   tggtaccagc agctcccagg 20
<210> 323
   <211> 39
   <212> DNA
   <213> Artificial Sequence
<220>
   <223> Description of Artificial Sequence: Synthetic primer
<400> 323
   gcctccctcg cgccatcagt ggtaccagca gctcccagg 39
<210> 324
   <211> 20
   <212> DNA
   <213> Artificial Sequence
<220>
   <223> Description of Artificial Sequence: Synthetic primer
<400> 324
   tggtaccagc agcttccagg 20
<210> 325
   <211> 39
   <212> DNA
   <213> Artificial Sequence
<220>
   <223> Description of Artificial Sequence: Synthetic primer
<400> 325
   gcctccctcg cgccatcagt ggtaccagca gcttccagg 39
<210> 326
   <211> 20
   <212> DNA
   <213> Artificial Sequence
<220>
   <223> Description of Artificial Sequence: Synthetic primer
<400> 326
   ctgcactgga accagcagtg 20
<210> 327
   <211> 20
   <212> DNA
   <213> Artificial Sequence
<220>
   <223> Description of Artificial Sequence: Synthetic primer
<400> 327
   tctctggctc caagtctggc 20
<210> 328
   <211> 39
   <212> DNA
   <213> Artificial Sequence
<220>
   <223> Description of Artificial Sequence: Synthetic primer
<400> 328
   gcctccctcg cgccatcagt ctctggctcc aagtctggc 39
<210> 329
   <211> 20
   <212> DNA
   <213> Artificial Sequence
<220>
   <223> Description of Artificial Sequence: Synthetic primer
<400> 329
   accagcagaa gccaggccag 20
<210> 330
   <211> 20
   <212> DNA
   <213> Artificial Sequence
<220>
   <223> Description of Artificial Sequence: Synthetic primer
<400> 330
   gaagccagga caggcccctg 20
<210> 331
   <211> 20
   <212> DNA
   <213> Artificial Sequence
<220>
   <223> Description of Artificial Sequence: Synthetic primer
<400> 331
   ctgagcgatt ctctggctcc 20
<210> 332
   <211> 39
   <212> DNA
   <213> Artificial Sequence
<220>
   <223> Description of Artificial Sequence: Synthetic primer
<400> 332
   gcctccctcg cgccatcagc tgagcgattc tctggctcc 39
<210> 333
   <211> 20
   <212> DNA
   <213> Artificial Sequence
<220>
   <223> Description of Artificial Sequence: Synthetic primer
<400> 333
   ttctctgggt ccacctcagg 20
<210> 334
   <211> 39
   <212> DNA
   <213> Artificial Sequence
<220>
   <223> Description of Artificial Sequence: Synthetic primer
<400> 334
   gcctccctcg cgccatcagt tctctgggtc cacctcagg 39
<210> 335
   <211> 20
   <212> DNA
   <213> Artificial Sequence
<220>
   <223> Description of Artificial Sequence: Synthetic primer
<400> 335
   ttctctggct ccagctcagg 20
<210> 336
   <211> 39
   <212> DNA
   <213> Artificial Sequence
<220>
   <223> Description of Artificial Sequence: Synthetic primer
<400> 336
   gcctccctcg cgccatcagt tctctggctc cagctcagg 39
<210> 337
   <211> 20
   <212> DNA
   <213> Artificial Sequence
<220>
   <223> Description of Artificial Sequence: Synthetic primer
<400> 337
   tcggtcaagc tcacctgcac 20
<210> 338
   <211> 39
   <212> DNA
   <213> Artificial Sequence
<220>
   <223> Description of Artificial Sequence: Synthetic primer
<400> 338
   gcctccctcg cgccatcagg ggctgaccgc tacctcacc 39
<210> 339
   <211> 20
   <212> DNA
   <213> Artificial Sequence
<220>
   <223> Description of Artificial Sequence: Synthetic primer
<400> 339
   cagcctgtgc tgactcagcc 20
<210> 340
   <211> 20
   <212> DNA
   <213> Artificial Sequence
<220>
   <223> Description of Artificial Sequence: Synthetic primer
<400> 340
   ccagccgctt ctctggatcc 20
<210> 341
   <211> 39
   <212> DNA
   <213> Artificial Sequence
<220>
   <223> Description of Artificial Sequence: Synthetic primer
<400> 341
   gcctccctcg cgccatcagc cagccgcttc tctggatcc 39
<210> 342
   <211> 20
   <212> DNA
   <213> Artificial Sequence
<220>
   <223> Description of Artificial Sequence: Synthetic primer
<400> 342
   ccatctcctg cacccgcagc 20
<210> 343
   <211> 20
   <212> DNA
   <213> Artificial Sequence
<220>
   <223> Description of Artificial Sequence: Synthetic primer
<400> 343
   tccccwggag ggacagtcac 20
<210> 344
   <211> 20
   <212> DNA
   <213> Artificial Sequence
<220>
   <223> Description of Artificial Sequence: Synthetic primer
<400> 344
   ctcmcctgca ccctgagcag 20
<210> 345
   <211> 20
   <212> DNA
   <213> Artificial Sequence
<220>
   <223> Description of Artificial Sequence: Synthetic primer
<400> 345
   agaccgccac actcacctgc 20
<210> 346
   <211> 20
   <212> DNA
   <213> Artificial Sequence
<220>
   <223> Description of Artificial Sequence: Synthetic primer
<400> 346
   ctgatcgstt ctctggctcc 20
<210> 347
   <211> 39
   <212> DNA
   <213> Artificial Sequence
<220>
   <223> Description of Artificial Sequence: Synthetic primer
<400> 347
   gcctccctcg cgccatcagc tgatcgsttc tctggctcc 39
<210> 348
   <211> 20
   <212> DNA
   <213> Artificial Sequence
<220>
   <223> Description of Artificial Sequence: Synthetic primer
<400> 348
   ctgcccggtt ctcaggctcc 20
<210> 349
   <211> 20
   <212> DNA
   <213> Artificial Sequence
<220>
   <223> Description of Artificial Sequence: Synthetic primer
<400> 349
   atccaggaag aggatgagag 20
<210> 350
   <211> 39
   <212> DNA
   <213> Artificial Sequence
<220>
   <223> Description of Artificial Sequence: Synthetic primer
<400> 350
   gcctccctcg cgccatcaga tccaggaaga ggatgagag 39
<210> 351
   <211> 20
   <212> DNA
   <213> Artificial Sequence
<220>
   <223> Description of Artificial Sequence: Synthetic primer
<400> 351
   ctccagcctg aggacgaggc 20
<210> 352
   <211> 39
   <212> DNA
   <213> Artificial Sequence
<220>
   <223> Description of Artificial Sequence: Synthetic primer
<400> 352
   gcctccctcg cgccatcagc tccagcctga ggacgaggc 39
<210> 353
   <211> 20
   <212> DNA
   <213> Artificial Sequence
<220>
   <223> Description of Artificial Sequence: Synthetic primer
<400> 353
   gctcccgggt agaagtcact 20
<210> 354
   <211> 20
   <212> DNA
   <213> Artificial Sequence
<220>
   <223> Description of Artificial Sequence: Synthetic primer
<400> 354
   agtgtggcct tgttggcttg 20
<210> 355
   <211> 39
   <212> DNA
   <213> Artificial Sequence
<220>
   <223> Description of Artificial Sequence: Synthetic primer
<400> 355
   gccttgccag cccgctcaga gtgtggcctt gttggcttg 39
<210> 356
   <211> 19
   <212> DNA
   <213> Artificial Sequence
<220>
   <223> Description of Artificial Sequence: Synthetic primer
<400> 356
   gcctccctcg cgccatcag 19
<210> 357
   <211> 19
   <212> DNA
   <213> Artificial Sequence
<220>
   <223> Description of Artificial Sequence: Synthetic primer
<400> 357
   gccttgccag cccgctcag 19
<210> 358
   <211> 20
   <212> DNA
   <213> Artificial Sequence
<220>
   <223> Description of Artificial Sequence: Synthetic primer
<400> 358
   gggctgaccg ctacctcacc 20

## Description

### FIELD OF THE INVENTION

The invention relates to a method to identify and characterise the immunoprofile of a patient and predict the likelihood of which disease the patient might have.

### BACKGROUND OF THE INVENTION

Scientists have known for a number of years that certain diseases are associated with particular genes or genetic mutations. Genetic causation, however, accounts for only a portion of the diseases diagnosed in humans. Many diseases appear to be linked in some way to the immune system's response to infectious and environmental agents, but how the immune system plays a role in diseases such as cancer, Alzheimer's, costochondritis, fibromyalgia, lupus, and other diseases is still being determined.

The human genome comprises a total number of 567-588 IG (immunoglobulin) and TR (T cell receptor) genes (339-354 IG and 228-234 TR) per haploid genome, localized in the 7 major loci. They comprise 405-418 V, 32 D, 105-109 J and 25-29 C genes. The number of functional IG and TR genes is 321-353 per haploid genome. They comprise 187-216 V, 28 D, 86-88 J and 20-21 C genes (http://imgt.cines.fr). Through rearrangement of these genes, an estimated 2.5x10⁷ possible antibodies or T cell receptors can be generated.

A few diseases to date have been associated with the body's reaction to a common antigen (Prinz, J. et al., Eur. J. Immunol. (1999) 29(10): 3360-3368, "Selection of Conserved TCR VDJ Rearrangements in Chronic Psoriatic Plaques Indicates a Common Antigen in Psoriasis Vulgaris) and/or to specific VDJ rearrangements (Tamaru, J. et al., Blood (1994) 84(3): 708-715, "Hodgkin's Disease with a B-cell Phenotype Often Shows a VDJ Rearrangement and Somatic Mutations in the VH Genes). What is needed is a better method for evaluating changes in human immune response cells and associating those changes with specific diseases.

WO2012/097374 discloses a method for identifying normal vs. abnormal immune status in an individual based on CDR3 sequencing data, wherein the diversity of clonotypes is analysed by calculating a diversity index (claim 4, [0007], [0015]).

### SUMMARY OF THE INVENTION

The invention is defined by the scope of the claims. Disclosed is a method for evaluating changes in immune response cell populations and associating those changes with a specific disease. In one aspect of the invention, the method comprises the steps of (a) isolating a subpopulation of white blood cells from at least one human or animal subject, (b) isolating RNA from the subpopulation of cells, (c) amplifying the RNA using RT-PCR in a first amplification reaction to produce amplicons using nested primers, at least a portion of the nested primers comprising additional nucleotides to incorporate into a resulting amplicon a binding site for a communal primer, (d) separating the amplicons from the first amplification reaction from one or more unused primers from the first amplification reaction, (e) amplifying, by the addition of communal primers in a second amplification reaction, the amplicons of the first amplification reaction having at least one binding site for a communal primer, and (f) sequencing the amplicons of the second amplification reaction to identify antibody and/or receptor rearrangements in the subpopulation of cells. In one embodiment, the subpopulation may comprise a whole blood population or another mixed population sample.

In one embodiment, the step of isolating a subpopulation of white blood cells may be performed by flow cytometry to separate naive B cells, mature B cells, memory B cells, naive T cells, mature T cells, and memory T cells. In various embodiments of the method, the recombinations in the subpopulation of cells are rearrangements of B-cell immunoglobulin heavy chain (IgH), kappa and/or lambda light chains (IgK, IgL), T-cell receptor Alpha, Beta, Gamma, Delta. The method may optionally comprise an additional step comprising (g) comparing the rearrangements identified for a population of individuals to whom a vaccine has been administered with the rearrangements identified for a population of individuals to whom the vaccine was not administered to evaluate the efficacy of the vaccine in producing an immune response.

The method may also optionally comprise the additional step of (g) comparing the rearrangements identified for a population of normal individuals with the rearrangements identified for a population of individuals who have been diagnosed with a disease to determine if there is a correlation between a specific rearrangement or set of rearrangements and the disease.

In various aspects, the method can produce semi-quantitative amplification of polynucleotides comprising complementarity determining region 3 (CDR3s), which result from genetic rearrangements within T or B cells and are responsible for the affinity and specificity of antibodies and/or T cell receptors for specific antigens. Semi-quantitative amplification provides a method to not only detect the presence of specific CDR3 sequences, but also determine the relative abundance of cells which have produced the necessary recombination events to produce those CDR3 sequences.

Further disclosed is a method for analyzing semi-quantitative sequence information to provide one or more immune status reports for a human or animal. The method for producing an immune status report comprising the steps of (a) identifying one or more distinct CDR3 sequences that are shared between a subject's immunoprofile and a cumulative immunoprofile from a disease library stored in a database, summing a total number of a subject's detected sequences corresponding to those shared distinct CDR3 sequences, and computing the percentage of the total number of detected sequences in the subject's immunoprofile that are representative of those distinct CDR3s shared between the subject's immunoprofile and the disease library to create one or more original sharing indices; (b) randomly selecting sequences from a public library stored in a database to form a sub-library, the sub-library comprising a number of sequences that is approximately equal to the number of distinct CDR3 sequences in the disease library, identifying one or more distinct CDR3 sequences that are shared between the subject's immunoprofile and the sub-library, summing a total number of detected sequences corresponding to those shared CDR3 sequences, and calculating a percentage of the total number of detected sequences in the subject's immunoprofile that are shared between the subject's immunoprofile and the sub-library to create a sampling sharing index; (c) repeating step (b) at least 1000 or more times; and (d) estimating the P-value as the fraction of times the sampling sharing indices are greater than or equal to the original sharing index between a patient's immunoprofile and a disease library.

### BRIEF DESCRIPTION OF THE DRAWINGS

The disclosure can be better understood with reference to the following drawings. The elements of the drawings are not necessarily to scale relative to each other, emphasis instead being placed upon clearly illustrating the principles of the disclosure. Furthermore, like reference numerals designate corresponding parts throughout the several views.
Figure 1a and Figure 1b are photographs of a gel illustrating the presence of amplification products obtained by the method disclosed herein using primers disclosed herein.
Figure 2a and Figure 2b are cartoons representing the observed difference in diversity between an immunoprofile in an individual with a disease and an individual who is generally healthy, with each filled circle representing a distinct CDR3 sequence and the size of the circle representing the number of times that the distinct CDR3 sequence is found in the immunoprofile.
Figure 3 is a diagram illustrating the method for generating a public library.
Figure 4 is a diagram illustrating the method for generating a disease library.
Figure 5 illustrates results obtained by comparing a patient immunoprofile with a disease library, calculating a percentage for each distinct CDR3 in the patient immunoprofile that is shared between the two, and adding those percentages to produce a sum, or sharing index.
Figure 6 illustrates results obtained by comparing a patient immunoprofile with a subset of a public library, calculating a percentage for each distinct CDR3 that is shared between the two, and adding those percentages in the patient immunoprofile to produce a sum, or sharing index.

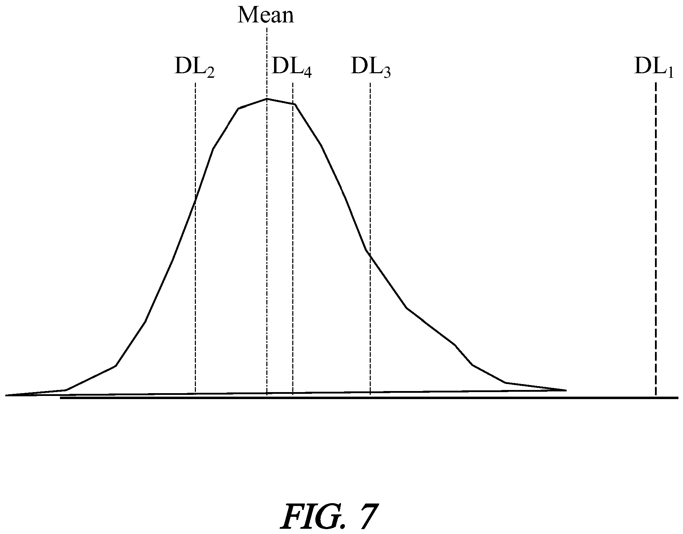

Figure 7 is a graph illustrating the method of the invention, where the area under the curve represents total sharing indices obtained for subsets of a public library (sub-libraries), a P-value is estimated, and sharing indices for comparisons of an individual's immunoprofile and one or more disease libraries are represented by vertical lines (DL₁, DL₂, etc.).

### DETAILED DESCRIPTION

The inventors have developed methods for evaluating antibody and T cell receptor rearrangements from a large number of cells, the methods being useful for comparing rearrangements identified in populations of individuals to determine whether there is a correlation between a specific rearrangement or set of rearrangements and a disease, or certain symptoms of a disease. The method is also useful for establishing a history of the immune response of an individual or individuals in response to infectious and/or environmental agents, as well as for evaluating the efficacy of vaccines.

Disclosed herein is a method for evaluating changes in immune response cell populations and associating those changes with a specific disease. In one aspect of the invention, the method comprises the steps of (a) isolating a subpopulation of white blood cells from at least one human or animal subject, (b) isolating RNA from the subpopulation of cells, (c) amplifying the RNA using RT-PCR in a first amplification reaction to produce amplicons using nested primers, at least a portion of the nested primers comprising additional nucleotides to incorporate into a resulting amplicon a binding site for a communal primer, (d) separating the amplicons from the first amplification reaction from one or more unused primers from the first amplification reaction, (e) amplifying, by the addition of communal primers in a second amplification reaction, the amplicons of the first amplification reaction having at least one binding site for a communal primer, and (f) sequencing the amplicons of the second amplification reaction to identify antibody and/or receptor rearrangements in the subpopulation of cells. In one embodiment, the subpopulation may comprise a whole blood population or another mixed population sample.

In one embodiment, a peripheral blood sample is taken from a patient and the step of isolating a subpopulation of white blood cells may be performed by flow cytometry to separate naive B cells, mature B cells, memory B cells, naïve T cells, mature T cells, and memory T cells. In various embodiments of the method, the recombinations in the subpopulation of cells are rearrangements of B-cell immunoglobulin heavy chain (IgH), kappa and/or lambda light chains (IgK, IgL), T-cell receptor Beta, Gamma, or Delta.

In a second aspect, the method may comprise an additional step (g) comparing the rearrangements identified for a population of normal individuals with the rearrangements identified for a population of individuals who have been diagnosed with a disease to determine if there is a correlation between a specific rearrangement or set of rearrangements and the disease.

In another aspect, the method may comprise an additional step comprising (g) comparing the rearrangements identified for a population of individuals to whom a vaccine has been administered with the rearrangements identified for a population of individuals to whom the vaccine was not administered to evaluate the efficacy of the vaccine in producing an immune response.

In some embodiments, the step of separating the amplicons from the first amplification reaction from one or more unused primers from the first amplification reaction may be omitted and the two amplification reactions may be performed in the same reaction tube.

The inventor previously developed a PCR method known as tem-PCR, which has been described in publication number WO2005/038039. More recently, the inventor has developed a method called arm-PCR, which was described in WO2009/124293. Also described is an apparatus for detecting target polynucleotides in a sample, the apparatus comprising a first amplification chamber for thermocycling to amplify one or more target polynucleotides to produce amplicons using nested primers, at least a portion of the nested primers comprising additional nucleotides to incorporate into a resulting amplicon a binding site for a communal primer; a means for separating the amplicons from the first amplification reaction from one or more unused primers from the first amplification reaction; and a second amplification chamber for thermocycling to amplify one or more amplicons produced during the first amplification reaction by the addition of communal primers in a second amplification reaction, the amplicons of the first amplification reaction having at least one binding site for at least one communal primer.

Also described is a PCR chip comprising a first PCR chamber fluidly connected to both a waste reservoir and a second PCR chamber, the waste reservoir and second PCR chamber each additionally comprising at least one electrode, the electrodes comprising a means for separating amplicons produced from the first PCR chamber. The second PCR chamber is fluidly connected to a hybridization and detection chamber, the hybridization and detection chamber comprising microspheres, or beads, arranged so that the physical position of the beads is an indication of a specific target polynucleotide's presence in the sampled analyzed by means of the chip.

The tem-PCR, and especially the arm-PCR, methods provide semi-quantitative amplification of multiple polynucleotides in one reaction. Additionally, arm-PCR provides added sensitivity. Both provide the ability to amplify multiple polynucleotides in one reaction, which is beneficial in the present method because the repertoire of various T and B cells, for example, is so large. The addition of a communal primer binding site in the amplification reaction, and the subsequent amplification of target molecules using communal primers, gives a quantitative, or semi-quantitative result-making it possible to determine the relative amounts of the cells comprising various rearrangements within a patient blood sample. Clonal expansion due to recognition of antigen results in a larger population of cells which recognize that antigen, and evaluating cells by their relative numbers provides a method for determining whether an antigen exposure has influenced expansion of antibody-producing B cells or receptor-bearing T cells. This is helpful for evaluating whether there may be a particular population of cells that is prevalent in individuals who have been diagnosed with a particular disease, for example, and may be especially helpful in evaluating whether or not a vaccine has achieved the desired immune response in individuals to whom the vaccine has been given.

There are several commercially available high throughput sequencing technologies, such as Roche Life Sciences's 454 sequencing. In the 454 sequencing method, 454A and 454B primers are linked onto PCR products either during PCR or ligated on after the PCR reaction. When done in conjunction with tem-PCR or arm-PCR, 454A and 454B primers may be used as communal primers in the amplification reactions. PCR products, usually a mixture of different sequences, are diluted to about 200 copies per µl. In an "emulsion PCR" reaction, (a semisolid gel like environment) the diluted PCR products are amplified by primers (454A or 454B) on the surface of the microbeads. Because the PCR templates are so dilute, usually only one bead is adjacent to one template, and confined in the semisolid environment, amplification only occurs on and around the beads. The beads are then eluted and put onto a plate with specially designed wells. Each well can only hold one bead. Reagents are then added into the wells to carry out pyrosequencing. A fiber-optic detector may be used to read the sequencing reaction from each well and the data is collected in parallel by a computer. One such high throughput reaction could generate up to 60 million reads (60 million beads) and each read can generate about 300bp sequences.

One aspect disclosed herein involves the development of a database of "personal immunorepertoires," or immunoprofiles, so that each individual may establish a baseline and follow the development of immune responses to antigens, both known and unknown, over a period of years. This information may, if information is gathered from a large number of individuals, provide an epidemiological database that will produce valuable information, particularly in regard to the development of those diseases, such as cancer and heart disease, which are thought to often arise from exposure to viral or other infectious agents or transformed cells, many of which have as yet been unidentified. One particularly important use for the method disclosed herein involves the evaluation of children to determine whether infectious disease, environmental agents, or vaccines may be the cause of autism. For example, many have postulated that vaccine administration may trigger the development of autism. However, many also attribute that potential correlation to the use of agents such as thimerosol in the vaccine, and studies have demonstrated that thimerosol does not appear to be a causative agent of the disease. There is still speculation that the development of cocktail vaccines has correlated with the rise in the number of cases of autism, however, gathering data to evaluate a potential causal connection for multiple antigens is extremely difficult. The method disclosed herein simplifies that process and may provide key information for a better understanding of autism and other diseases in which the immune response of different individuals may provide an explanation for the differential development of disease in some individuals exposed to an agent or a group of agents, while others similarly exposed do not develop the disease.

Imbalances of the immunoprofile, triggered by infection, may lead to many diseases, including cancers, leukemia, neuronal diseases (Alzheimer's, Multiple Sclerosis, Parkinson's, autism etc.), autoimmune diseases, and metabolic diseases. These diseases may be called immunoprofile diseases. There may be two immunoprofile disease forms. (1) a "loss of function" form, and (2) a "gain of function" form. In the "loss of function" form, a person is susceptible to a disease because his/her restricted and/or limited immunoprofile lacks the cells that produce the most efficient and necessary IGs and TRs. In the "gain of function" form, a person is susceptible to a disease because his/her immunoprofile gained cells that produce IGs and TRs that normally should not be there. In the "loss of a function" (LOF) immunoprofile diseases, an individual does not have the appropriate functional B or T cells to fight a disease. His/her HLA typing has determined that those cells are eliminated during the early stages of the immune cell maturation process, the cells generally being eliminated because they react to strongly to his/her own proteins.

Further disclosed is a method comprising (a) amplifying and sequencing one or more RNAs from the T cells and/or B cells from one or more individuals, (b) inputting the sequences into a database to provide data which may be stored on a computer, server, or other electronic storage device, (c) inputting identifying information and characteristics for an individual corresponding to the sequences of the one or more RNAs as data which may also be stored on a computer, server, or other electronic storage device, and (d) evaluating the data of step (b) and step (e) for one or more individuals to determine whether a correlation exists between the one or more RNA sequences and one or more characteristics of the individual corresponding to the sequence(s). Identifying information may include, for example, a patient identification number, a code comprising the patient's HLA type, a disease code comprising one or more clinical diagnoses that may have been made, a "staging code" comprising the date of the sample, a cell type code comprising the type of cell subpopulation from which the RNA was amplified and sequenced, and one or more sequence codes comprising the sequences identified for the sample.

The described method includes a novel primer design that not only allows amplification of the entire immunorepertoire, but also allows amplification in a highly multiplex fashion and semiquantitatively. Multiplex amplification requires that only a few PCR or RT-PCR reactions will be needed. For example, all IGs may be amplified in one reaction, or it could be divided into two or three reactions for IgH, IgL or IgK. Similarly, the T-cell receptors (TRs) may be amplified in just one reaction, or may be amplified in a few reactions including TRA, TRB, TRD, and TRG. Semi-quantitative amplification means that all the targets in the multiplex reaction will be amplified independently, so that the end point analysis of the amplified products will reflect the original internal ratio among the targets.

In various aspects, the method can produce semi-quantitative amplification of polynucleotides comprising complementarity determining regions (CDRs), which result from genetic rearrangements within T or B cells and are responsible for the affinity and specificity of antibodies and/or T cell receptors for specific antigens. Semi-quantitative amplification provides a method to not only detect the presence of specific CDR3 sequences, but also determine the relative numbers of cells which have produced the necessary recombination events to produce those CDR3 sequences.

Further disclosed is a method for analyzing semi-quantitative sequence information to provide one or more immune status reports for a human or animal. The method for producing an immune status report comprising the steps of (a) identifying one or more distinct CDR3 sequences that are shared between a subject's immunoprofile and a disease library stored in a database, summing the total of those shared CDR3 sequences and computing the percentage of the total number of sequences in the subject's immunoprofile that are shared between the subject's immunoprofile and the disease library to create one or more original sharing indices; (b) randomly selecting sequences from a public library stored in a database to form a sub-library, the sub-library comprising a number of sequences that is approximately equal to the number of distinct sequences in the disease library, identifying one or more distinct CDR3 sequences that are shared between the subject's immunoprofile and the sub-library, summing the total of those shared CDR3 sequences and calculating the percentage of the total number of sequences in the subject's immunoprofile that are shared between the subject's immunoprofile and the sub-library to create a sampling sharing index; (c) repeating step (b) at least 1000 or more times; and (d) estimating the P-value as the fraction of times the sampling sharing indices are greater than or equal to the original sharing index between a patient's immunoprofile and a disease library.

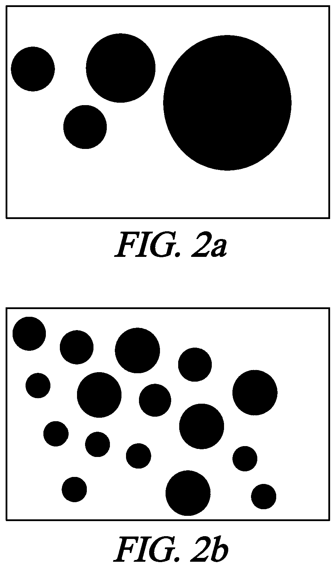

The inventors have discovered that the immunoprofile of individuals who have certain diseases, such as, for example, cancer, autoimmune disease, etc., may be characterized by a lack of diversity in one or more immune cell population(s). Figure 2 is a cartoon illustrating the difference that may be observed between, for example, the distinct type and number of T-cells present in a blood sample from a cancer patient (Fig. 2a) and a healthy patient (Fig. 2b), where each circle represents a distinct type of T-cell, as represented by an amplified and sequenced recombined cDNA of the complementarity determining region of the T-cell receptor (e.g., CDR3), and the relative number of cells which are determined, by PCR amplification and sequencing, to share the same CDR3 sequence. As Fig. 2 a indicates, there may be fewer distinct cells of different specificities, but larger numbers of cells of certain specificities, as represented by the CDR3 sequences. Fig. 2b illustrates a normal profile of more different cells, but fewer numbers of each type of cell sharing the same CDR3 sequence.

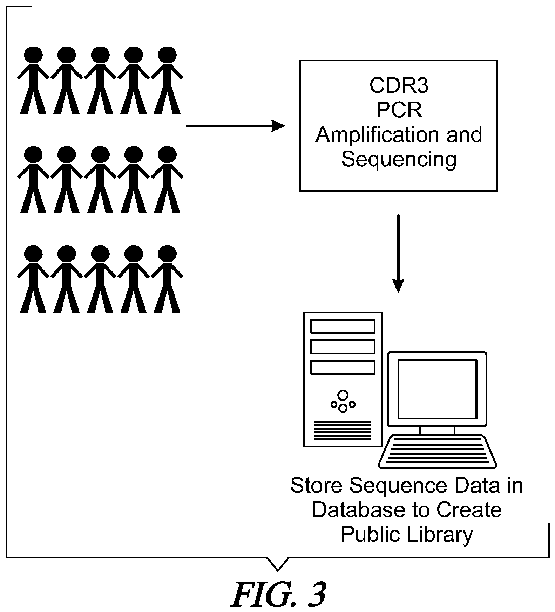

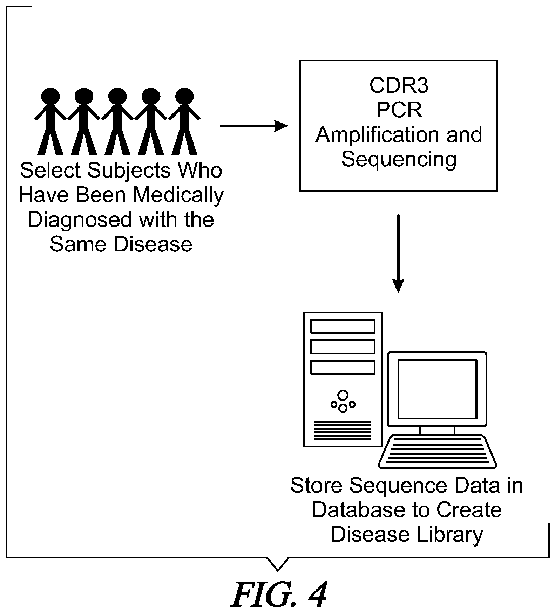

The list of each distinct CDR3-expressing cell, and the numbers of such cells represented within a blood or tissue sample from a human or animal, can constitute an immunoprofile for that human or animal. Compiling the immunoprofiles from a group of humans, for example, the group comprising both healthy individuals and individuals with various different diseases may provide a "public library" that is representative of the type of diversity found in a normal population (Fig. 3). Similarly, compiling the immunoprofiles of a group of individuals who have been clinically diagnosed with a particular disease may provide a "disease library" that is representative of the lack of diversity, the specific CDR3s of the expanded populations of cells, etc. (Fig. 4). These immunoprofiles may be stored in a database, accessible via computer access to the internet, for example, so that the information may be used in the method of the invention to analyze the immune status of a patient.

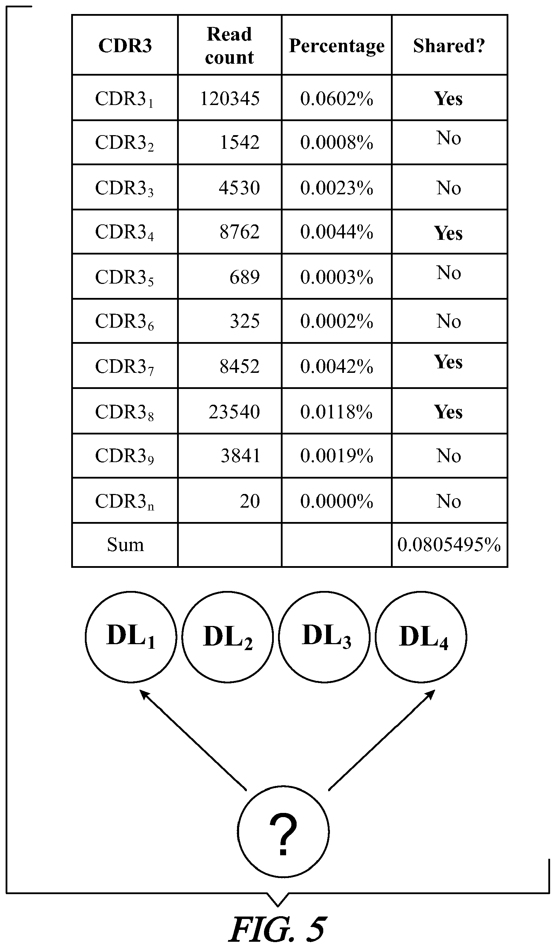

An immunoprofile, comprising a listing of distinct CDR3-expressing cells ("distinct CDR3s", those cells sharing a unique CDR3 sequence) and the numbers of each distinct CDR3 present in a blood or tissue sample from an individual may be produced for an individual patient. The patient's immunoprofile is compared to the combined immunoprofiles of a group of patients who have been diagnosed with a particular disease (a disease library, stored in a database). This can be done for a series of disease libraries, and shown in Fig. 5.

Millions of possible combinations are possible for the public library, the immune systems of most of those individuals generally exhibiting increased diversity over that of a group of individuals who have been diagnosed with a specific disease. Therefore, the inventors determined that an accurate assessment and comparison for the method of the invention would be facilitated by the step of preparing sub-libraries by randomly sampling/selecting from the lists of distinct CDR3s and their numbers in the public library. The number of distinct CDR3s, represented by unique peptide sequence of CDR3 fragments, should be approximately equal to the number of distinct CDR3s identified in the disease library, or an average calculated from more than one disease library. Producing a significant number of sub-libraries, such as, for example, 1000 or more sub-libraries, produced by randomly sampling from the public library, increases the presence of a variety of distinct CDR3s and produces a result that is statistically significant effective for identifying and characterizing an individual patient's immunoprofile as normal ("healthy") or characterized by the presence of a type and number of cells that have been associated with a particular disease.

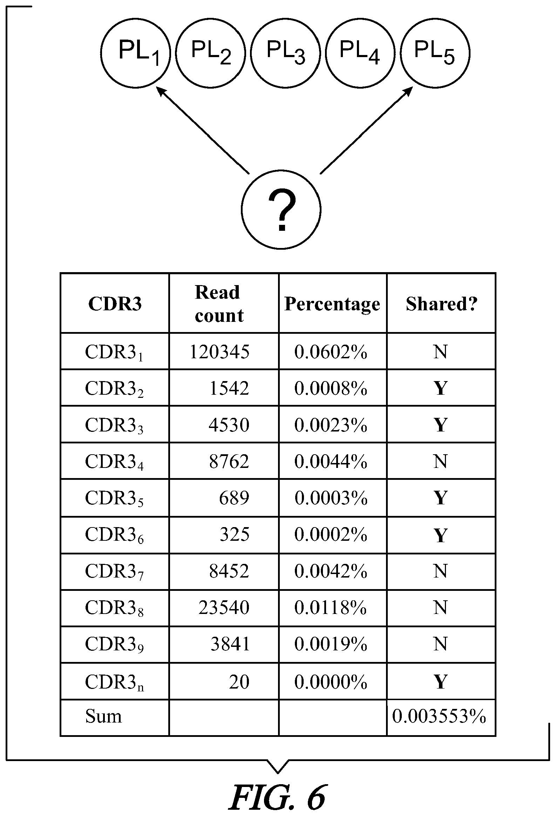

In the method disclosed herein, a patient supplies a clinical sample comprising, for example, blood or tissue, from which distinct CDR3s are semi-quantitatively amplified and sequenced. This provides the identity and the relative abundance of each CDR3 for all distinct CDR3s. This information may be entered into a program which accesses a database containing at least one public library and one or more disease libraries. Software used for data entry and/or analysis may be accessed via internet access to the database, or may be located on an individual personal computer, with internet access to the sequence information in the database. Comparisons are obtained between the individual immunoprofile and the various libraries and sub-libraries, and results are generated as generally illustrated in Fig. 5 and Fig. 6, where specific CDR3 sequences are detected, the numbers of those distinct CDR3 sequences detected are counted, and a determination is made as to whether or not that specific distinct CDR3 is present in both the individual's immunoprofile and a specific library (i.e., that specific distinct CDR3 is "shared" between the individual and the library). The percentages representing numbers of those CDR3s that are determined to be shared are added together to produce a sum comprising the fraction of the total that comprises CDR3s in the individual's immunoprofile shared between the individual's immunoprofile and the specific library (i.e., a "sharing index"). From the results obtained for the sub-libraries, a P-value is calculated as the probability that a random percentage would be greater than or equal to the percentage noted for a particular disease library, and a significant result is noted when the fraction of times the sampling sharing indices exceeds the original sharing index for a particular library is less than 0.01, for instance. If that sharing index represents the relationship between the individual's immunoprofile and a disease library, the individual may then be informed of the likelihood that the individual/patient has the disease represented by the specific disease library. If P-values computed against all disease libraries is greater than 0.01, the individual's report may indicate that the immune profile looks normal and the disease state has not been detected.

As sequence data is compiled and stored in one or more databases for multiple populations of individuals, it may additionally be possible to associate certain sharing indexes with libraries representing populations with pre-conditions or predispositions to certain diseases. The immune system is both proactive and reactive, and changes in the immune system, reflected in the immunoprofile, may provide the first-and sometimes the only-signal that a predisposition, a precondition, or even an established disease is present. The inventors have utilized the method to demonstrate that certain types of cancers, inflammatory bowel disease, and certain viral infections may be detected by determining the sharing index between a patient and an established disease library, obtained by sequencing CDR3s using the ARM-PCR method to produce a subset of the immunorepertoire representing the CDR3s present.

The results are even more reliable when a filter is applied to the sequence data. For example, the inventors have developed a "SMART" filter for the sequence data that aids in the generation of significantly more reliable results. This is described further in the Examples.

By way of further explanation, the following example may be illustrative of the methods disclosed herein. Blood samples may be taken from children prior to administration of any vaccines, those blood samples for each child establishing a "baseline" from which future samples may be evaluated. For each child, the future samples may be utilized to determine whether there has been an exposure to an agent which has expanded a population of cells known to be correlated with a disease, and this may serve as a "marker" for the risk of development of the disease in the future. Individuals so identified may then be more closely monitored so that early detection is possible, and any available treatment options may be provided at an earlier stage in the disease process.

By means of providing another example, blood samples may be taken from children prior to administration of any vaccines, those blood samples from each child establishing a "baseline" from which future samples may be evaluated. For each child and for the entire population of children in the study, those baselines may be compared to the results of RNA sequencing of T and B cells using target-specific primers to amplify antibody and T-cell receptor, after vaccine administration. The comparison may further involve the evaluation of data regarding symptoms, diagnosed diseases, and other information associated for each individual with the corresponding antibody, and T-cell receptor sequences. If a relationship exists between the administration of a vaccine and the development of a particular disease, individuals who exhibit symptoms of that disease may also share a corresponding antibody or T-cell receptor, for example, or a set of corresponding antibodies or T-cell receptors.

The method disclosed herein may be especially useful for identifying commonalities between individuals with autoimmune diseases, for example, and may provide epidemiological data that will better describe the correlation between infectious and environmental factors and diseases such as heart disease, atherosclerosis, diabetes, and cancer-providing "biomarkers" that signal either the presence of a disease, or the tendency to develop disease.

The method may also be useful for development passive immunity therapies. For example, following exposure to an infectious agent, certain antibody-producing B cells and/or T cells are expanded. The method enables the identification of protective antibodies, for example, and those antibodies may be utilized to provide passive immunity therapies in situations where such therapy is needed.

The method may also provide the ability to accomplish targeted removal of cells with undesirable rearrangements, the method providing a means by which such cells rearrangements may be identified.

The inventor has identified and developed target-specific primers for use in the method of the invention. T-cell-specific primers are shown in Table 1, and antibody-specific primers are shown in Table 2. An additional embodiment disclosed herein is a method of using any one or a combination of primers of Table 1 or Table 2, to amplify RNA from a blood sample, and more particularly to identify antibodies, T-cell receptors, and HLA molecules within a population of cells.

Arm-PCR or tem-PCR may be used to amplify genes coding for the immunoglobulin superfamily molecules in am amplification method described previously by the inventor (Han et al., 2006. Simultaneous Amplification and Identification of 25 Human Papillomavirus Types with Templex Technology. J. Clin. Micro. 44(11). 4157-4162). In a tem-PCR reaction, nested gene-specific primers are designed to enrich the targets during initial PCR cycling. Later, universal "Super" primers are used to amplify all targets. Primers are designated as Fₒ (forward out), Fᵢ (forward in), Rᵢ (reverse in), Rₒ (reverse out), FS (forward super primer) and RS,(reverse super primer), with super primers being common to a variety of the molecules due to the addition of a binding site for those primers at the end of a target-specific primer. The gene-specific primers (Fₒ, Fᵢ, Rᵢ, and Rₒ) are used at extremely low concentrations. Different primers are involved in the tem-PCR process at each of the three major stages. First, at the "enrichment" stage, low-concentration gene-specific primers are given enough time to find the templates. For each intended target, depending on which primers are used, four possible products may be generated: Fₒ/Rₒ, Fᵢ/Rₒ, Fᵢ/Rᵢ, and Fₒ/Rᵢ. The enrichment stage is typically carried out for 10 cycles. In the second, or "tagging" stage, the annealing temperature is raised to 72°C, and only the long 40-nucleotide inside primers (Fᵢ and Rᵢ) will work. After 10 cycles of this tagging stage, all PCR products are "tagged" with the universal super primer sequences. Then, at the third "amplification" stage, high-concentration super primers work efficiently to amplify all targets and label the PCR products with biotin during the process. Specific probes may be covalently linked with Luminex color-coated beads.

To amplify the genes coding for immunoglobulin superfamily molecules, the inventor designed nested primers based on sequence information in the public domain. For studying B and T cell VDJ rearrangement, the inventor designed primers to amplify rearranged and expressed RNAs. Generally, a pair of nested forward primers is designed from the V genes and a set of reverse nested primers are designed from the J or C genes. The average amplicon size is 250-350bp. For the IgHV genes, for example, there are 123 genes that can be classified into 7 different families, and the present primers are designed to be family specific. However, if sequencing the amplified cDNA sequences, there are enough sequence diversities to allow further differentiation among the gene within the same family. For the MHC gene locus, the intent is to amplify genomic DNA.

### EXAMPLES

### Calculation of Sharing Index

Assuming that **S** is a subject's immunoprofile (IP), which is represented by **N** unique CDR3 sequences CDR3₁, CDR3₂, ... CDR3ₙ, each CDR3 has its own frequency **s₁, s₂, ... sₙ.**

**D** is a disease library, which is the sum of a certain number of patients' immunoprofile with **M** unique CDR3s. All patients in the disease library were diagnosed to have the same disease.

**P** is a public library, which is the sum of a large number of control's immunoprofile.

The **S**haring **I**ndex is defined as the sum of **sₓ, s_{y}, ... s_{z}**, where CDR3ₓ, CDR3_{y}, ... CDR3_{z} are shared in the subject's immunoprofile and a library. Note that **sₓ, s_{y},** ... **s_{z}** is the frequency of CDR3s in the subject's immunoprofile, not in the library.

Assuming that there are always more unique CDR3s in a public library (P) than in a disease library (D), **M** unique CDR3s in the public library are randomly selected and used to create a sub-library P1 and the sharing index (**SIₚ₁**) between the subject and the sub-library computed according to above formula. The sampling procedure is repeated 1000 or more times and 1000 or more **SIₚₓ** are computed.

The sharing index **SI_{d}** between the subject and the disease library are computed in the same manner. The **P-value** is defined as the fraction of all **SI**s (**SIₚ₁**, **SIₚ₂,** ... **SIₚₓ, SI_{d},** (Note that **SI_{d}** is included), which is equal to or greater than **SI_{d}**. Note that, when sampling CDR3s in the public library, CDR3s found in **x** control's immunoprofiles are given **x** times of chances to be sampled.

### Amplification of T or B Cell Rearrangement Sites

All oligos were resuspended using 1x TE. All oligos except 454A and 454B were resuspended to a concentration of 100pmol/µL 454A and 454B were resuspended to a concentration of 1000pmol/µL 454A and 454B are functionally the same as the communal primers described previously, the different sequences were used for follow up high throughput sequencing procedures.

Three different primer mixes were made. An Alpha Delta primer mix included 82 primers (all of TRAV-C + TRDV-C), a Beta Gamma primer mix included 79 primers (all of TRBVC and TRGV-C) and a B cell primer mix that included a total of 70 primers. Fₒ, Fᵢ, and Rᵢ primers were at a concentration of 1pmol/µL. Rₒ primers were at a concentration of 5 pmol/µL. 454A and 454B were at a concentration of 30 pmol/µL.

Three different RNA samples were ordered from ALLCELLS (www.allcells.com). All samples were diluted down to a final concentration of 4 ng/uL. The samples ordered were:

| **Cell type:** | **Source:** |
|---|---|
| ALL-PB-MNC | A patient with acute lymphoblastic leukemia |
| NPB-Pan T Cells | Normal T cells |
| NPB-B Cells | Normal B cells |

RT-PCR was performed using a Qiagen One-Step RT-PCR kit. Each sample contained the following:
10 µL of Qiagen Buffer
2 µL of DNTP's
2 µl of Enzyme
23.5 µL of dH₂O
10 µL of the appropriate primer mix
2.5 µL of the appropriate template (10ng of RNA total)

The samples were run using the following cycling conditions:
50°C for 30 minutes
95°C for 15 minutes
94°C for 30 seconds
15 cycles of
55°C for 1 minute
72°C for 1 minute
94°C for 15 seconds
6 cycles of
70°C for 1 minute 30 seconds
94°C for 15 seconds
30 cycles of
55°C for 15 seconds
72°C for 15 seconds
72°C for 3 minutes
4°C Hold

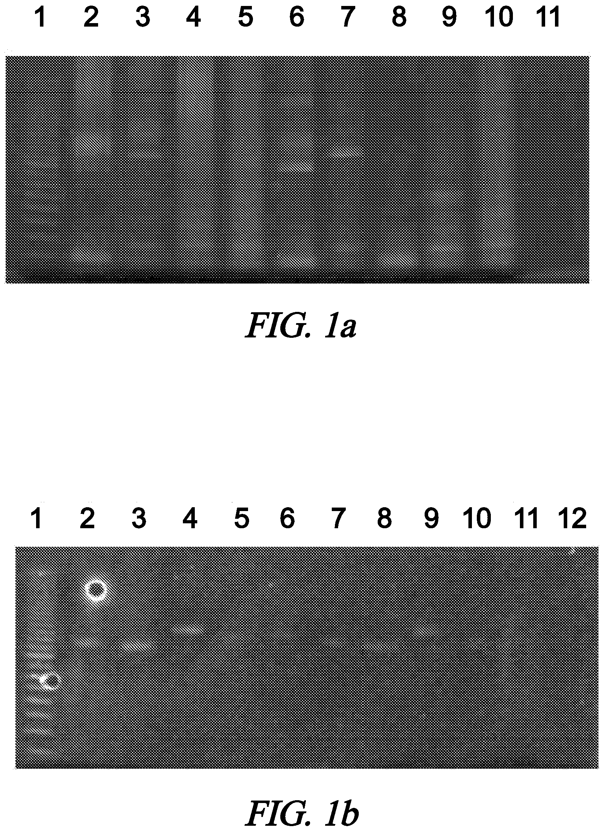

The order of samples placed in the gel shown in Fig. 1a was: (1) Ladder (500bp being the largest working down in steps of 20bp, the middle bright band in Fig. 1a is 200bp); (2) α + δ primer mix with 10ng Pan T Cells Template; (3) β + γ primer mix with 10ng Pan T Cells Template; (4) B Cell primer mix with 10ng B Cells Template; (5) B Cell primer mix with 10ng ALL Cells Template; (6) α + δ primer mix with 10ng ALL Cells Template; (7) β + γ primer mix with 10ng ALL Cells Template; 8. α + δ primer mix blank; (9) β + γ primer mix blank; (10) B Cell primer mix blank; (11)Running buffer blank. These samples were run on a pre-cast ClearPAGE® SDS 10% gel using 1X ClearPAGE® DNA native running buffer.

The initial experiment showed that a smear is generated from PCR reactions where templates were included. The smears indicate different sizes of PCR products were generated that represented a mixture of different VDJ rearrangements. There is some background amplification from the B cell reaction. Further improvement on that primer mix was required to clean up the reaction.

To determine whether the PCR products indeed include different VDJ rearrangements, it was necessary to isolate and sequence the single clones. Instead of using the routine cloning procedures, the inventor used a different strategy. PCR products generated from the Alpha Delta mix and the Beta Gamma mix (lanes 2 and 3 in Fig. 1 a) were diluted 1:1000 and a 2 µl aliquot used as PCR template in the following reaction. Then, instead of using a mixture of primers that targeting the entire repertoire, one pair of specific Fi and Ri primers were used (5 pmol each) to amplify only one specific PCR product. The following cycling conditions were used to amplify the samples:
95°C for 5 minutes
30 cycles of
94°C for 30 seconds
72°C for 1 minute
72°C for 3 minutes
4°C hold

A Qiagen PCR kit was used to amplify the products. The Master Mix used for the PCR contained the following:

| | Per Reaction | Master Mix x 12 |
|---|---|---|
| 10x PCR Buffer | 5µL | 60µL |
| dNTP | 1µL | 12µL |
| HotStartTaq Plus | 0.25µL | 3µL |
| H₂O | 39.75 µL | 477 µL |

The photograph of the gel in Fig. 1b shows the PCR products of the following reactions: (1) Ladder; (2) TRAV1Fi+TRACRi with alpha delta Pan T PCR product; (3) TRAV2Fi+TRACRi with alpha delta Pan T PCR product; (4) TRAV3Fᵢ+TRACRᵢ with alpha delta Pan T PCR product; (5) TRAV4Fᵢ+TRACRᵢ with alpha delta Pan T PCR product; (6) TRAV5Fᵢ+TRACRᵢ with alpha delta Pan T PCR product; (7) TRAV1Fᵢ+TRACRᵢ with alpha delta Pan T PCR product; (8) TRAV2Fᵢ+TRACRᵢ with alpha delta Pan T PCR product; (9) TRAV3Fᵢ+TRACRᵢ with alpha delta Pan T PCR product; (10) TRAV4Fᵢ+TRACRᵢ with alpha delta Pan T PCR product; (11) TRAV5Fᵢ+TRACRᵢ with alpha delta Pan T PCR product; (12) PCR Blank. Primers listed as F₁ are "forward inner" primers and primers listed as Fₒ are "forward outer" primers, with Rᵢ and Rₒ indicating "reverse inner" and "reverse outer" primers, respectively.

As illustrated by Fig. 1b, a single PCR product was generated from each reaction. Different size bands were generated from different reactions. This PCR cloning approach is successful for two major reasons-(1) The PCR templates used in this reaction were diluted PCR products (1:1000) of previous reactions that used primer mixes to amplify all possible VDJ rearrangements (for example, a primer mix was used that included total of 82 primers to amplify T cell receptor Alpha and Delta genes) and (2) Only one pair of PCR primers, targeting a specific V gene, are used in each reaction during this "cloning" experiment. Some of these products were gel purified and sequenced. The following are example sequences obtained from the protocol described above. In every case, a single clone was obtained, and a specific T cell receptor V gene that matched the Fi primer was identified.
TRAV1 template + 454A as sequencing primer:
TRAV1 template + 454A as sequencing primer:
TRAV2 template + 454A as sequencing primer:
TRAV3 template + 454A as sequencing primer:
TRAV4 template + 454A as sequencing primer:
TRAV5 template + 454A as sequencing primer:
TRBV19Fi template + 454A as sequencing primer:
TRBV20Fi template + 454A as sequencing primer:
TRBV21Fi template + 454A as sequencing primer:
TRBV23Fi template + 454A as sequencing primer:
TRBV24Fi template + 454A as sequencing primer:

To investigate the impact of artifacts on the overall repertoire analysis of the TCRβ transcriptome, the inventors conducted control experiments using chemically synthesized TCRβ CDR3 templates. For this, the inventors chemically synthesized four distinct clones, clonally purified each clone, and prepared different mixes of the four constructs as templates for amplicon rescue multiplex (ARM)-PCR. Two different reaction mixtures were subjected to two independent ARM-PCR reactions, and the pooled PCR products were sequenced at a length of 100bp from both ends using the Illimuna HiSeq2000®. The inventors first joined together paired-end reads through overlapping alignment with a modified Needleman-Wunsch algorithm, and then mapped the merged sequences to germline V, D and J reference sequences.

Without cleaning, the inventors obtained a total of 5,729,613 sequences from template mix I that could be mapped to TCRβ V, D and J segments. Surprisingly, the sequence reads purportedly represented a total of 36,439 unique CDR3 variants. Therefore, given that only four distinct CDR3 variants were present in the template mixtures, virtually all of the identified CDR3 variants must be non-authentic. Similar results were obtained for the second template mix, in which a total of 9,131,681 VDJ-mapped sequences were identified that mimicked the existence of 50,354 unique TCRβ CDR3 variants. The inventors' independent sequencing experiments show that only a few distinct CDR3 template variants can create artifactual repertoire diversities that far outweigh the real template diversity, and thus the inventors set out to eliminate these artifacts.

The quality of 3' end Illumina sequencing reads is generally considered to be low. In the context of repertoire sequencing, this is troublesome because PCR primers need to be positioned distal enough from the hypervariable V(D)J junctions to avoid negative effects due to primer-template mismatching. As a consequence, the CDR3 segments of interest are generally "shifted" closer to the 3' end of the sequencing reads, the region with increased sequencing error rates. Another technical issue that deserves attention is the observation that sequencing errors are context-specific and consequently strand-specific. Therefore, it is realistic to assume that the probability that a sequencing error in a forward read coincides with that in the corresponding reverse read is rare.

Considering this, the inventors devised a paired-end strategy that affords double-strand sequencing of complete TCR CDR3 segments on the basis of the Illumina® technology. In this approach, forward and reverse sequencing primers are positioned at the framework region 3 and at the TCR J region or the 5' end of the C region, respectively. Taking into account the average length of Illumina sequence reads (currently 100-150 bp) this design enables the complete sequencing of both strands that define a CDR3 segment. In a second step, the forward and reverse reads are then analyzed for sequence mismatches and CDR3 sequences that exhibit non-identity of both strands are eliminated using a newly developed paired-end filtering algorithm.

Applying this sequencing error filter to the 5,729,613 CDR3 sequences obtained for template mix I, the inventors identified a total of 2,751,131 (48%) CDR3 sequences that contained conflicting sequence information on their opposite strands. Discarding of these sequences resulted in the elimination of 35,455 (97.2%) distinct artifactual CDR3 variants. Consistent with this, the paired-end filter removed 4,308,020 (47%) CDR3 sequences from template mix II, leading to the elimination of 49,063 (97.4%) artifactual CDR3 variants. A total of 973 and 1271 unique CDR3 variants, respectively, passed through the filter. These results indicate that paired-end sequencing and filtering reduces the total number of non-authentic unique CDR3 sequences by almost two orders of magnitude.

Detailed analysis of the frequency distribution of the non-authentic CDR3 variants after the sequencing error filter revealed that in both mixtures approximately 50% of all artifacts were single-copy sequences. About 10% of these artifactual CDR3s displayed >100 copy numbers and accounted for > 80% of all artifactual CDR3 variants. Given that variable TCR genes do not undergo somatic hypermutation, the inventors developed a reference algorithm that identifies and removes CDR3 sequence reads that display nucleotide mismatches relative to the mapped germline V, D and J reference sequences, as these must be artifacts generated at the level of PCR amplification or sequencing.

Applying this filtering algorithm to the "paired-end filtered" sequences of template mix I, a total of 29,804 sequences, which corresponded to 609 unique CDR3 variants, were removed. For template mix II, 54,516 artifactual sequences (831 unique CDR3 variants) were identified. Thus, the use of the reference sequence filter leads to a 60% reduction of non-authentic distinct CDR3 sequences. The reference filter is ineffective at the V-J and D-J junctions because the randomly added nucleotides in these regions during somatic recombination cannot be mapped. Therefore, the inventors implemented a PCR filter after computational simulation experiments to better understand four variables: the impact of the initial template number, the replication efficiency of each cycle, the cycle number (n), and the DNA polymerase error rate (µ) on the total end-point error rate. In contrast, the inventors noted that the PCR polymerase error rate has a pronounced effect on the number of accumulated errors

In the inventors' control sequencing experiments, PCR amplification was performed with 15 cycles and 45 cycles in the first and second reaction, using Taq polymerase. To simulate error accumulation during the ARM-PCR reactions more realistically, the PCR efficiency was set to decreased 5% per cycle for the first 25 cycles and 10% per cycle for the remaining cycles. The PCR efficiency was reset to 1.0 for each fresh PCR reaction. Furthermore, the inventors allowed mutation at the second position. Published substitution error rates for Taq enzyme, expressed as errors per bp per cycle, range from 0.023 x10⁻⁴ to 2.1 x10⁻⁴. In the simulation experiments, the substitution error rate was set at 2.7 x10⁻⁵, and the insertion-deletion (indel) error rate was set as 1.0 x10⁻⁶. Taq polymerase is known to have a much higher insertion-and-deletion (indel) mutation rate in homopolymeric region of templates. For a homopolymeric region, indel mutation in any position of this region generates identical pattern. Therefore, the indel error rate in a homopolymeric region was set as n x µ, where n is the length of the homopolymeric region and µ is 1.0 x 10⁻⁶.

Because the impact of the initial template number and the PCR efficiency on the endpoint error rate is small, it should be safe to apply the same end-point error rate estimated from the simulation experiments to molecules with different initial number and different replication efficiencies in a multiplex PCR reaction. The cutoff error rates (µ) were empirically set as error rates at the 9999th 10000-quantiles point for each category. For two similar CDR3 sequences, A and B, of frequency NA and NB (NA >> NB) that differ in less than three positions, if NA ^{∗} µ ≥ NB, where µ is the corresponding cutoff error rate, CDR3 sequence B will be excluded. Applying this filtering algorithm to the "reference filtered" sequences of template mix I, a total of 22,369 sequences, which corresponded to 281 unique CDR3 variants, were removed. For template mix II, 39,920 artifactual sequences (348 unique CDR3 variants) were identified (Table 1). Thus, the use of the PCR amplification error filter leads to a further reduction of non-authentic distinct CDR3 sequences by around 80%.

In the pool of sequences that had passed through the above filters, the inventors identified several high-abundance CDR3 variants, which differed from their most similar input template sequences at multiple positions. Because the occurrence of PCR substitution and/or indel mutation at multiple positions of CDR3 fragments is extremely rare according to simulation experiments, those CDR3 variants must arise from other source of artifacts. Intriguingly, the inventors noted that some of these sequences were composed of the fragments of two distinct input templates and exhibited clear breakpoints, which identified them as chimeras. Chimeric sequences are PCR artifacts that arise from incomplete primer extension or template switching during PCR and form mosaic-like structures. In light of this unexpected PCR artifact, the inventors developed a computational "mosaic filter." Using this filtering algorithm, the inventors identified a total of 17 and 15 chimeric sequences in template mixtures I and II, respectively. Of note, some of these CDR3 chimeras displayed sequence copy numbers >1000, indicating that the inventors' algorithm for the filter is capable of identifying high-abundance chimeric CDR3 sequences.

Application of the filtering algorithms resulted in the elimination of 99.8% of the non-authentic unique CDR3 sequences generated by high-throughput sequencing of only four defined TCR CDR3 templates. Only 62 and 73 artifactual CDR3 sequences, respectively, passed through all filters. Among these, the two most abundant CDR3 sequences were identical in both mixing experiments. Most likely they represent chimeric artifacts which escaped filtering because of a single nucleotide substitution located exactly at the breakpoint. Among the remaining erroneous CDR3, 85% (n=53) and 75% (n=55) were single reads, respectively. To eliminate this minor fraction of artifacts, the inventors propose that high-stringency data analysis of TCR immune repertoires should include an additional filter that removes single copy CDR3 reads (frequency threshold filter).

**Table 1**

| **Locus** | **Primer Name** | **Sequence** | **SEQ ID NO.** | **Sequence** | **SEQ ID NO.** |
|---|---|---|---|---|---|
| | | | | | |
| TRAV-C | TRAV1Fo | | 12 | | 12 |
| | TRAV1Fi | | 13 | | 14 |
| | TRAV2Fo | | 15 | | 15 |
| | TRAV2Fi | | 16 | | 17 |
| | TRAV3Fo | | 18 | | 18 |
| | TRAV3Fi | | 19 | | 20 |
| | TRAV4Fo | | 21 | | 21 |
| | TRAV4Fi | | 22 | | 23 |
| | TRAV5Fo | | 24 | | 24 |
| | TRAV5Fi | | 25 | | 26 |
| | TRAV6Fo | | 27 | | 27 |
| | TRAV6Fi | | 28 | | 29 |
| | TRAV7Fo | | 30 | | 30 |
| | TRAV7Fi | | 31 | | 32 |
| | TRAV8Fo | | 33 | | 33 |
| | TRAV8Fi | | 34 | | 35 |
| | | | | | |
| | TRAV9Fo | | 36 | | 36 |
| | TRAV9Fi | | 37 | | 38 |
| | TRAV10Fo | | 39 | | 39 |
| | TRAV10Fi | | 40 | | 41 |
| | TRAV11Fo | | 42 | | 42 |
| | TRAV11Fi | | 43 | | 44 |
| | TRAV12Fo | | 45 | | 45 |
| | TRAV12Fi | | 46 | | 47 |
| | TRAV13Fo | | 48 | | 48 |
| | TRAV13Fi | | 49 | | 50 |
| | TRAV14Fo | | 51 | | 51 |
| | TRAV14Fi | | 52 | | 53 |
| | TRAV16Fo | | 54 | | 54 |
| | TRAV16Fi | | 55 | | 56 |
| | TRAV17Fo | | 57 | | 57 |
| | TRAV17Fi | | 58 | | 59 |
| | | | | | |
| | TRAV18Fo | | 60 | | 60 |
| | TRAV18Fi | | 61 | | 62 |
| | TRAV19Fo | | 63 | | 63 |
| | TRAV19Fi | | 64 | | 65 |
| | TRAV20Fo | | 66 | | 66 |
| | TRAV20Fi | | 67 | | 68 |
| | TRAV21Fo | | 69 | | 69 |
| | TRAV21Fi | | 70 | | 71 |
| | TRAV22Fo | | 72 | | 72 |
| | TRAV22Fi | | 73 | | 74 |
| | TRAV23Fo | | 75 | | 75 |
| | TRAV23Fi | | 76 | | 77 |
| | TRAV24Fo | | 78 | | 78 |
| | TRAV24Fi | | 79 | | 80 |
| | TRAV25Fo | | 81 | | 81 |
| | TRAV25Fi | | 82 | | 83 |
| | TRAV26Fo | | 84 | | 84 |
| | | | | | |
| | TRAV26Fi | | 85 | | 86 |
| | TRAV27Fo | | 87 | | 87 |
| | TRAV27Fi | | 88 | | 89 |
| | TRAV29Fo | | 90 | | 90 |
| | TRAV29Fi | | 91 | | 92 |
| | TRAV30Fo | | 93 | | 93 |
| | TRAV30Fi | | 94 | | 95 |
| | TRAV34Fo | | 96 | | 96 |
| | TRAV34Fi | | 97 | | 98 |
| | TRAV35Fo | | 99 | | 99 |
| | TRAV35Fi | | 100 | | 101 |
| | TRAV36Fo | | 102 | | 102 |
| | TRAV36Fi | | 103 | | 104 |
| | TRAV38Fo | | 105 | | 105 |
| | TRAV38Fi | | 106 | | 107 |
| | TRAV39Fo | | 108 | | 108 |
| | TRAV39Fi | | 109 | | 110 |
| | | | | | |
| | TRAV40Fo | | 111 | | 111 |
| | TRAV40Fi | | 112 | | 113 |
| | TRAV41Fo | | 114 | | 114 |
| | TRAV41Fi | | 115 | | 116 |
| | | | | | |
| | TRACRo | | 117 | | 117 |
| | TRACRi | | 118 | | 119 |
| | | | | | |
| TRBV-C | TRBV1Fo | | 120 | | 120 |
| | TRBV1Fi | | 121 | | 122 |
| | TRBV2Fo | | 123 | | 123 |
| | TRBV2Fi | | 124 | | 125 |
| | TRBV3Fo | | 126 | | 126 |
| | TRBV3Fi | | 127 | | 128 |
| | TRBV4Fo | | 129 | | 129 |
| | TRBV4Fi | | 130 | | 131 |
| | TRBV5Fo | | 132 | | 132 |
| | TRBV5aFi | | 133 | | 134 |
| | TRBV5bFi | | 135 | | 136 |
| | TRBV6aFo | | 137 | | 137 |
| | TRBV6bFo | | 138 | | 138 |
| | TRBV6aF | | 139 | | 140 |
| | TRBV6bFi | | 141 | | 142 |
| | TRBV7Fo | | 143 | | 143 |
| | TRBV7aFi | | 144 | | 145 |
| | TRBV7bFi | | 146 | | 147 |
| | TRBV7cFi | | 148 | | 149 |
| | TRBV7dFi | | 150 | | 151 |
| | TRBV9Fo | | 152 | | 152 |
| | TRBV9Fi | | 153 | | 154 |
| | TRBV10Fo | | 155 | | 155 |
| | TRBV10Fi | | 156 | | 157 |
| | TRBV11Fo | | 158 | | 158 |
| | | | | | |
| | TRBV11Fi | | 159 | | 160 |
| | TRBV12Fo | | 161 | | 161 |
| | TRBV12aFi | | 162 | | 163 |
| | TRBV12bFi | | 164 | | 165 |
| | TRBV13Fo | | 166 | | 166 |
| | TRBV13Fi | | 167 | | 168 |
| | TRBV14Fo | | 169 | | 169 |
| | TRBV14Fi | | 170 | | 171 |
| | TRBV15Fo | | 172 | | 172 |
| | TRBV15Fi | | 173 | | 174 |
| | TRBV16Fo | | 175 | | 175 |
| | TRBV16Fi | | 176 | | 177 |
| | TRBV17Fo | | 178 | | 178 |
| | TRBV17Fi | | 179 | | 180 |
| | TRBV18Fo | | 181 | | 181 |
| | TRBV18Fi | | 182 | | 183 |
| | TRBV19Fo | | 184 | | 184 |
| | TRBV19Fi | | 185 | | 186 |
| | TRBV20Fo | | 187 | | 187 |
| | TRBV20Fi | | 188 | | 189 |
| | TRBV21Fo | | 190 | | 190 |
| | TRBV21 Fi | | 191 | | 192 |
| | TRBV23Fo | | 193 | | 193 |
| | TRBV23Fi | | 194 | | 195 |
| | TRBV24Fo | | 196 | | 196 |
| | TRBV24Fi | | 197 | | 198 |
| | TRBV25Fo | | 199 | | 199 |
| | TRBV25Fi | | 200 | | 201 |
| | TRBV26Fo | | 202 | | 202 |
| | TRBV26Fi | | 203 | | 204 |
| | TRBV27Fo | | 205 | | 205 |
| | TRBV27Fi | | 206 | | 207 |
| | TRBV28Fo | | 208 | | 208 |
| | TRBV28Fi | | 209 | | 210 |
| | | | | | |
| | TRBV29Fo | | 211 | | 211 |
| | TRBV29Fi | | 212 | | 213 |
| | TRBV30Fo | | 214 | | 214 |
| | TRBV30Fi | | 215 | | 216 |
| | | | | | |
| | TRBCRo | | 217 | | 217 |
| | TRBCRi | | 218 | | 219 |
| | | | | | |
| TRDV-C | TRDV1 Fo | | 220 | | 220 |
| | TRDV1 Fi | | 221 | | 222 |
| | TRDV2Fo | | 223 | | 223 |
| | TRDV2Fi | | 224 | | 225 |
| | TRDV3Fo | | 226 | | 226 |
| | TRDV3Fi | | 227 | | 228 |
| | | | | | |
| | TRDCRo | | 229 | | 229 |
| | TRDCRi | | 230 | | 231 |
| TRGV-C | TRGV1-5Fo | | 232 | | 232 |
| | TRGV1-5,8Fi | | 233 | | 234 |
| | TRGV8Fo | | 235 | | 235 |
| | TRGV5pFi | | 236 | | 237 |
| | TRGV9Fo | | 238 | | 238 |
| | TRGV9Fi | | 239 | | 240 |
| | TRGV10Fo | | 241 | | 241 |
| | TRGV10Fi | | 242 | | 243 |
| | TRGV11Fo | | 244 | | 244 |
| | TRGV11Fi | | 245 | | 246 |
| | | | | | |
| | TRGCRo | | 247 | | 247 |
| | TRGCRi | | 248 | | 249 |

**Table 2**

| **Locus** | **Primer Name** | **Sequence** | **SEQ ID NO.** | **Ordered** | **SEQ ID NO.** |
|---|---|---|---|---|---|
| | | | | | |
| **IgHV-J** | IgHV1aFo | | 250 | | 250 |
| | IgHV1bFo | | 251 | | 251 |
| | IgHV1aFi | | 252 | | 253 |
| | IgHV1bFi | | 254 | | 255 |
| | IgHV1cFi | | 256 | | 257 |
| | IgHV1dFi | | 258 | | 259 |
| | IgHV2Fo | | 260 | | 260 |
| | IgHV2Fi | | 261 | | 262 |
| | IgHV3aFo | | 263 | | 263 |
| | IgHV3bFo | | 264 | | 264 |
| | IgHV3cFo | | 265 | | 265 |
| | IgHV3dFo | | 266 | | 266 |
| | IgHV3aFi | | 267 | | 268 |
| | IgHV3bFi | | 269 | | 270 |
| | IgHV3cFi | | 271 | | 272 |
| | IgHV3dFi | | 273 | | 274 |
| | IgHV3eFi | | 275 | | 276 |
| | IgHV4Fo | | 277 | | 277 |
| | IgHV4Fi | | 278 | | 279 |
| | IgHV5Fo | | 280 | | 280 |
| | IgHV5Fi | | 281 | | 282 |
| | IgHV6Fo | | 283 | | 283 |
| | IgHV6Fi | | 284 | | 285 |
| | IgHV7Fo | | 286 | | 286 |
| | IgHV7Fi | | 287 | | 288 |
| | | | | | |
| | IgHJRo | | 289 | | 289 |
| | IgHJ1Ri | | 290 | | 291 |
| | IgHJ2Ri | | 292 | | 293 |
| | IgHJ3Ri | | 294 | | 295 |
| | IgHJ4Ri | | 296 | | 297 |
| | IgHJ5Ri | | 298 | | 299 |
| | IgHJ6Ri | | 300 | | 301 |
| | | | | | |
| **IgKV-C** | IgKV1Fo | | 302 | | 302 |
| | IgKV1Fi | | 303 | | 304 |
| | IgKV2Fo | | 305 | | 305 |
| | IgKV2aFi | | 306 | | 307 |
| | IgKV2bFi | | 308 | | 309 |
| | IgKV3-7Fo | | 310 | | 310 |
| | IgKV3Fi | | 311 | | 312 |
| | IgKV6-7Fi | | 313 | | 314 |
| | I g KV4-5Fi | | 315 | | 316 |
| | | | | | |
| | IgKCRo | | 317 | | 317 |
| | IgKCRi | | 318 | | 319 |
| | | | | | |
| **IgLV-C** | Ig LV1aFo | | 320 | | 320 |
| | IgLVIbFo | | 321 | | 321 |
| | ILV1aFi | | | | 323 |
| | IgLV1bFi | | 324 | | 325 |
| | IgLV2Fo | | 326 | | 326 |
| | IgLV2Fi | | 327 | | 328 |
| | IgLV3aFo | | 329 | | 329 |
| | IgLV3bFo | | 330 | | 330 |
| | IgLV3aFi | | 331 | | 332 |
| | IgLV3bFi | | 333 | | 334 |
| | IgLV3cFi | | 335 | | 336 |
| | IgLV4Fo | | 337 | | 337 |
| | IgLV4Fi | | 358 | | 338 |
| | IgLV5Fo | | 339 | | 339 |
| | IgLV5Fi | | 340 | | 341 |
| | IgLV6Fo | | 342 | | 342 |
| | IgLV7-8Fo | | 343 | | 343 |
| | IgLV9,11Fo | | 344 | | 344 |
| | IgLV10Fo | | 345 | | 345 |
| | IgLV6,8Fi | | 346 | | 347 |
| | IgLV7Fi | | 348 | | 348 |
| | | | | | |
| | IgLV9Fi | | 349 | | 350 |
| | IgLV10-11Fi | | 351 | | 352 |
| | | | | | |
| | IgLC1-7Ro | | 353 | | 353 |
| | IgLC1-7Ri | | 354 | | 355 |
| | | | | | |
| | 454A | | 356 | | 356 |
| | | | | | |
| | 454B | | 357 | | 357 |

## Claims

1. A computer-implemented method for analysing semi-quantitative sequence information to identify and characterise the immunoprofile of a human or animal subject as normal or as being likely to indicate the presence of a disease, the method comprising the steps of:
(a) identifying one or more distinct complementarity determining region 3 (CDR3) sequences that are shared between the subject's immunoprofile and a cumulative immunoprofile from a disease library representing a specific disease stored in a database;
(b) summing a total number of the subject's detected sequences corresponding to those shared distinct CDR3 sequences;
(c) computing the percentage of the total number of detected sequences in the subject's immunoprofile that are representative of those distinct CDR3s shared between the subject's immunoprofile and the disease library to create one or more original sharing indices;
(d) randomly selecting sequences from a public library stored in a database to form a sub-library, the sub-library comprising a number of distinct CDR3 sequences that is approximately equal to the number of distinct CDR3 sequences in the disease library;
(e) identifying one or more distinct CDR3 sequences that are shared between the subject's immunoprofile and the sub-library;
(f) summing a total number of detected sequences corresponding to those shared CDR3 sequences and calculating a percentage of the total number of detected sequences in the subject's immunoprofile that are shared between the subject's immunoprofile and the sub-library to create a sampling sharing index;
(g) repeating steps (d)-(f) for a number of times sufficient to produce a result that is statistically significant for identifying and characterising the immunoprofile of said subject as normal or as being likely to indicate the presence of a disease;
(h) estimating the P-value as the fraction of times the sampling sharing indices are greater than or equal to the original sharing index between the immunoprofile of said subject and the disease library; and
(i) characterising the immunoprofile of the subject as normal if the estimated P-value is greater than 0.01, or characterising the immunoprofile of the subject as being likely to indicate the presence of the disease represented by the disease library if the estimated P-value is less than 0.01.

2. The method of claim 1, wherein step (g) comprises repeating steps (d)-(f) at least 1000 or more times.

3. The method of claim 1 or claim 2, wherein step (a) is preceded by semi-quantitative amplification and sequencing of distinct CDR3s from said subject, wherein said semi-quantitative amplification and sequencing comprising the steps of:
(i) isolating a subpopulation of white blood cells from said subject;
(ii) isolating RNA from the subpopulation of cells;
(iii) amplifying the RNA using RT-PCR in a first amplification reaction to produce amplicons using nested primers, at least a portion of the nested primers comprising additional nucleotides to incorporate into a resulting amplicon a binding site for a communal primer;
(iv) separating the amplicons from the first amplification reaction from one or more unused primers from the first amplification reaction;
(v) amplifying, by the addition of communal primers in a second amplification reaction, the amplicons of the first amplification reaction having at least one binding site for a communal primer, wherein the product of the second amplification reaction is polynucleotides comprising distinct CDR3s; and
(vi) sequencing the amplicons of the second amplification reaction to identify antibody and/or receptor rearrangements in the subpopulation of cells;
wherein the amplification and sequencing of steps (v) and (vi) provide semi-quantitative sequence information for distinct CDR3s from the subject.

4. The method of claim 3, wherein the step of isolating a subpopulation of white blood cells is performed by flow cytometry.

5. The method of claim 3, wherein the subpopulation of white blood cells comprises T cells.

6. The method of claim 5, wherein the T cells are selected from the group consisting of naive T cells, mature T cells and memory T cells.

7. The method of claim 3, wherein the subpopulation of white blood cells comprises B cells.

8. The method of claim 7, wherein the B cells are selected from the group consisting of naive B cells, mature B cells and memory B cells.

9. The method of claim 3, wherein the rearrangements in the subpopulations of cells are selected from the group consisting of rearrangements of B-cell immunoglobulin heavy chain (IgH), B-cell kappa, B-cell lambda light chains, T-cell receptor Beta, T-cell Gamma and T-cell Delta.

## Patentansprüche

1. Computerimplementiertes Verfahren zum Analysieren semi-quantitativer Sequenzinformation, um das Immunprofil eines humanen oder tierischen Subjekts als normal oder als wahrscheinlich zu identifizieren und zu charakterisieren, um das Vorhandensein einer Krankheit anzuzeigen, wobei das Verfahren die folgenden Schritte umfasst:
(a) Identifizieren einer oder mehrerer unterschiedlicher, eine Komplementarität bestimmender Region 3 (CDR3)-Sequenzen, die zwischen dem Immunprofil des Subjekts und einem kumulativen Immunprofil aus einer Krankheitsbibliothek, die eine in einer Datenbank gespeicherte spezifische Krankheit repräsentiert, gemeinsam genutzt werden;
(b) Summieren einer Gesamtzahl der erfassten Sequenzen des Subjekts, die diesen gemeinsam genutzten unterschiedlichen CDR3-Sequenzen entsprechen;
(c) Berechnen des Prozentsatzes der Gesamtzahl von erfassten Sequenzen in dem Immunprofil des Subjekts, die repräsentativ für diese unterschiedlichen CDR3 sind, die zwischen dem Immunprofil des Subjekts und der Krankheitsbibliothek gemeinsam genutzt werden, um einen oder mehrere ursprüngliche gemeinsam genutzte Indizes zu erzeugen;
(d) zufälliges Auswählen von Sequenzen aus einer öffentlichen Bibliothek, die in einer Datenbank gespeichert ist, um eine Unterbibliothek zu bilden, wobei die Unterbibliothek eine Anzahl von unterschiedlichen CDR3-Sequenzen umfasst, die annähernd gleich der Anzahl von unterschiedlichen CDR3-Sequenzen in der Krankheitsbibliothek ist;
(e) Identifizieren einer oder mehrerer unterschiedlicher CDR3-Sequenzen, die zwischen dem Immunprofil des Subjekts und der Unterbibliothek gemeinsam genutzt werden;
(f) Summieren einer Gesamtzahl der erfassten Sequenzen, die diesen gemeinsam genutzten CDR3-Sequenzen entsprechen, und Berechnen eines Prozentsatzes der Gesamtzahl der erfassten Sequenzen im Immunprofil des Subjekts, die zwischen dem Immunprofil des Subjekts und der Unterbibliothek gemeinsam genutzt werden, um einen Index der gemeinsam genutzten Probenahmen zu erstellen;
(g) Wiederholen der Schritte (d)-(f) für eine Anzahl von Malen, die ausreicht, um ein Ergebnis zu erzeugen, das statistisch signifikant ist, um das Immunprofil des Probanden als normal oder als wahrscheinlich zu identifizieren und zu charakterisieren, um das Vorhandensein einer Krankheit anzuzeigen;
(h) Schätzen des P-Wertes als den Bruchteil der Male, bei denen die Indizes der gemeinsam genutzten Probenahmen größer oder gleich dem ursprünglichen Index einer gemeinsamen Nutzung zwischen dem Immunprofil des Subjekts und der Krankheitsbibliothek sind; und
(i) Charakterisieren des Immunprofils des Subjekts als normal, wenn der geschätzte P-Wert größer als 0,01 ist, oder Charakterisieren des Immunprofils des Subjekts als wahrscheinlich, um das Vorhandensein der Krankheit, die durch die Krankheitsbibliothek repräsentiert wird, anzuzeigen, wenn der geschätzte P-Wert kleiner als 0,01 ist.

2. Verfahren nach Anspruch 1, wobei der Schritt (g) Wiederholen der Schritte (d)-(f) zumindest 1000 oder mehr Male umfasst.

3. Verfahren nach Anspruch 1 oder Anspruch 2, wobei Schritt (a) eine semi-quantitative Amplifikation und Sequenzierung von unterschiedlichen CDR3s aus dem Subjekt vorausgeht, wobei die semi-quantitative Amplifikation und Sequenzierung die Schritte umfasst des:
(i) Isolierens einer Subpopulation von weißen Blutkörperchen aus dem Subjekt;
(ii) Isolierens von RNS aus der Subpopulation von Zellen;
(iii) Amplifizierens der RNS unter Verwendung von RT-PCR in einer ersten Amplifikationsreaktion zur Herstellung von Amplikons unter Verwendung von verschachtelten Primern, wobei mindestens ein Teil der verschachtelten Primer zusätzliche Nukleotide umfasst, um in ein resultierendes Amplikon eine Bindungsstelle für einen Gemeinschaftsprimer einzubauen;
(iv) Separierens der Amplikons aus der ersten Amplifikationsreaktion von einem oder mehreren nicht verwendeten Primern aus der ersten Amplifikationsreaktion;
(v) Amplifizierns der Amplikons der ersten Amplifikationsreaktion, die mindestens eine Bindungsstelle für einen Gemeinschaftsprimer aufweisen, durch die Zugabe von Gemeinschaftsprimern in einer zweiten Amplifikationsreaktion, wobei das Produkt der zweiten Amplifikationsreaktion Polynukleotide sind, die unterschiedliche CDR3s umfassen; und
(vi) Sequenzierens der Amplikone der zweiten Amplifikationsreaktion, um Antikörper- und/oder Rezeptor-Neuanordnungen in der Subpopulation von Zellen zu identifizieren;
wobei die Amplifikation und Sequenzierung der Schritte (v) und (vi) eine semi-quantitative Sequenzinformation für unterschiedliche CDR3s des Subjekts bereitstellen.

4. Verfahren nach Anspruch 3, wobei der Schritt des Isolierens einer Subpopulation von weißen Blutkörperchen durch Durchflusszytometrie durchgeführt wird.

5. Verfahren nach Anspruch 3, wobei die Subpopulation von weißen Blutkörperchen T-Zellen umfasst.

6. Verfahren nach Anspruch 5, wobei die T-Zellen ausgewählt sind aus der Gruppe bestehend aus naiven T-Zellen, reifen T-Zellen und Gedächtnis-T-Zellen.

7. Verfahren nach Anspruch 3, wobei die Subpopulation von weißen Blutkörperchen B-Zellen umfasst.

8. Verfahren nach Anspruch 7, wobei die B-Zellen ausgewählt sind aus der Gruppe bestehend aus naiven B-Zellen, reifen B-Zellen und B-Gedächtniszellen.

9. Verfahren nach Anspruch 3, wobei die Neuanordnungen in den Subpopulationen der Zellen ausgewählt sind aus der Gruppe bestehend aus Neuanordnungen der schweren B-Zellen-Immunglobulinkette (IgH), Kappa-B-Zellen, leichten B-Zellen-Lambdaketten, Beta-T-Zell-Rezeptor, Gamma-T-Zellen und Delta-T-Zellen.

## Revendications

1. Procédé implémenté par ordinateur d'analyse d'informations de séquence semi-quantitatives pour identifier et caractériser l'immunoprofil d'un sujet humain ou animal comme normal ou comme étant susceptible d'indiquer la présence d'une maladie, le procédé comprenant les étapes de :
(a) identification d'une ou plusieurs séquences de région déterminant la complémentarité 3 (CDR3) distinctes qui sont partagées entre l'immunoprofil du sujet et un immunoprofil cumulé issu d'une banque de maladies représentant une maladie spécifique stockée dans une base de données ;
(b) la sommation d'un nombre total des séquences détectées du sujet correspondant à ces séquences CDR3 distinctes partagées ;
(c) le calcul du pourcentage du nombre total de séquences détectées dans l'immunoprofil du sujet qui sont représentatives de ces CDR3 distinctes partagées entre l'immunoprofil du sujet et la banque de maladies pour créer un ou plusieurs indices de partage originaux ;
(d) la sélection aléatoire de séquences issues d'une banque publique stockée dans une base de données pour former une sous-banque, la sous-banque comprenant un nombre de séquences CDR3 distinctes qui est approximativement égal au nombre de séquences CDR3 distinctes dans la banque de maladies ;
(e) l'identification d'une ou plusieurs séquences CDR3 distinctes qui sont partagées entre l'immunoprofil du sujet et la sous-banque ;
(f) la sommation d'un nombre total de séquences détectées correspondant à ces séquences CDR3 partagées et le calcul d'un pourcentage du nombre total de séquences détectées dans l'immunoprofil du sujet qui sont partagées entre l'immunoprofil du sujet et la sous-banque pour créer un indice de partage d'échantillonnage ;
(g) la répétition des étapes (d) à (f) pendant un nombre de fois suffisant pour produire un résultat qui est statistiquement significatif pour identifier et caractériser l'immunoprofil dudit sujet comme normal ou comme étant susceptible d'indiquer la présence d'une maladie ;
(h) l'estimation de la valeur P comme la fraction de fois où les indices de partage d'échantillonnage sont supérieurs ou égaux à l'indice de partage original entre l'immunoprofil dudit sujet et la banque de maladies ; et
(i) la caractérisation de l'immunoprofil du sujet comme normal si la valeur P estimée est plus grande que 0,01, ou la caractérisation de l'immunoprofil du sujet comme étant susceptible d'indiquer la présence de la maladie représentée par la banque de maladies si la valeur P estimée est plus petite que 0,01.

2. Procédé selon la revendication 1, dans lequel l'étape (g) comprend la répétition des étapes (d) à (f) au moins 1000 fois ou plus.

3. Procédé selon la revendication 1 ou la revendication 2, dans lequel l'étape (a) est précédée par une amplification semi-quantitative et un séquençage de CDR3 distinctes à partir dudit sujet, dans lequel ladite amplification semi-quantitative et ledit séquençage comprennent les étapes :
(i) d'isolement d'une sous-population de globules blancs provenant dudit sujet ;
(ii) d'isolement de l'ARN issu de la sous-population de cellules ;
(iii) d'amplification de l'ARN à l'aide d'une RT-PCR dans une première réaction d'amplification pour produire des amplicons à l'aide d'amorces imbriquées, au moins une portion des amorces imbriquées comprenant des nucléotides additionnels pour incorporer dans un amplicon résultant un site de liaison pour une amorce commune ;
(iv) de séparation des amplicons issus de la première réaction d'amplification d'une ou plusieurs amorces inutilisées issues de la première réaction d'amplification ;
(v) d'amplification, par addition d'amorces communes dans une seconde réaction d'amplification, les amplicons de la première réaction d'amplification ayant au moins un site de liaison pour une amorce commune, dans lequel le produit de la seconde réaction d'amplification est les polynucléotides comprenant des CDR3 distinctes ; et
(vi) de séquençage des amplicons de la seconde réaction d'amplification pour identifier des réarrangements d'anticorps et/ou de récepteurs dans la sous-population de cellules ;
dans lequel l'amplification et le séquençage des étapes (v) et (vi) fournissent des informations de séquences semi-quantitatives pour des CDR3 distinctes provenant du sujet.

4. Procédé selon la revendication 3, dans lequel l'étape d'isolement d'une sous-population de globules blancs est réalisée par cytométrie en flux.

5. Procédé selon la revendication 3, dans lequel la sous-population de globules blancs comprend des cellules T.

6. Procédé selon la revendication 5, dans lequel les cellules T sont choisies dans le groupe consistant en les cellules T naïves, les cellules T matures et les cellules T mémoire.

7. Procédé selon la revendication 3, dans lequel la sous-population de globules blancs comprend des cellules B.

8. Procédé selon la revendication 7, dans lequel les cellules B sont choisies dans le groupe consistant en les cellules B naïves, les cellules B matures et les cellules B mémoire.

9. Procédé selon la revendication 3, dans lequel les réarrangements dans les sous-populations de cellules sont choisis dans le groupe consistant en des réarrangements de chaîne lourde d'immunoglobuline de cellules B (IgH), chaînes légères kappa de cellules B, lambda de cellules B, récepteur Bêta de cellules T, Gamma de cellules T et Delta de cellules T.
